# EUROPÄISCHE PATENTSCHRIFT

(11) **EP 3 536 383 B1**
(45) Veröffentlichungstag und Bekanntmachung des Hinweises auf die Patenterteilung: **27.09.2023**
(21) Anmeldenummer: 19020101.2
(22) Anmeldetag: 01.03.2019
(51) Int. Cl.: A61Q 5/02, A61Q 9/02, A61Q 11/00, A61Q 19/00, A61K 8/92

(54) **REINIGUNGS- UND PFLEGEZUBEREITUNGEN ENTHALTEND EIN POLYOXYALKYLEN CARBOXYLAT**
CLEANING AND CARE PREPARATIONS COMPRISING A POLYOXYALKYLENE CARBOXYLATE
PRÉPARATIONS DE NETTOYAGE ET DE SOIN COMPRENANT UN POLYOXYALKYLENE CARBOXYLATE

(30) Priorität: 06.03.2018 CH 2742018
(43) Veröffentlichungstag der Anmeldung: 11.09.2019
(73) Patentinhaber: Perfect Ideas GmbH, 6043 Adligenswil (CH)
(72) Erfinder:

(56) Entgegenhaltungen:
- EP-B1- 1 844 093
- WO-A1-2019/151800
- CH-A2- 705 757
- CN-A- 102 716 046
- CN-A- 103 356 427
- CN-A- 104 688 619
- DE-A1-102005 051 671
- KR-A- 20030 083 163
- US-A1- 2012 016 039
- US-A1- 2013 042 482
- US-B1- 9 005 674
- AMARI S ET AL: "Novel foaming agent derived from olive oil = Un nuevo espumante derivado del aceite de oliva", 1 January 1999 (1999-01-01), COMUNICACIONES PRESENTADAS A LAS XXIX JORNADAS DEL COMITÉ ESPAÑOL DE LA DETERGENCIA (CED), COMITÉ ESPANÕL DE LA DETERGENCIA, PAGE(S) 377 - 379, XP009518668, ISBN: 978-84-922519-3-3
- Reinol: "Reinolderm OLV4", , 1 September 2017 (2017-09-01), XP55721260, Retrieved from the Internet: URL:https://reinol.it/wp-content/uploads/2 017/09/OLV4-Product-Description.pdf [retrieved on 2020-04-01]
- ANONYMOUS: "Pores Refreshing Face Cleanser", GNPD, MINTEL, 1 July 2012 (2012-07-01), XP002769429,

## Beschreibung

### Gegenstand

Gegenstand dieser Anmeldung sind Reinigungs- und Pflegezubereitungen wie in den Ansprüchen definiert, die mindestens ein Polyoxyalkylen Carboxylat und weitere Tenside enthalten, sowie deren Verwendung als kosmetische oder medizinische Reinigungs- und Pflegemittel. Das oder die Polyoxyalkylen Carboxylate und das oder die zusätzlichen Tenside basieren auf Fettsäuren aus Pflanzenölen und weisen einen aussergewöhnlich hohen Anteil an langkettigen (≥ C17), mehrheitlich ungesättigten Kohlenwasserstoffketten auf.

### Problem - Stand der Technik

Die vorliegende Erfindung richtet sich auf eine Zubereitung mit verbesserter dermatologischer Verträglichkeit und geringerem Augenreizungspotential für die Verwendung der Mittel zur Körperreinigung und -pflege, der Haarwäsche, dem Abschminken, der Zahnpflege, und der Rasur; insbesondere für die Entfernung von Proteinablagerungen bzw. -verschmutzungen, sowie partikulärem Schmutz.

Die Entfernung von proteinhaltiger Verunreinigungen (Eiweissverunreinigungen) ist in der Körperpflege ausserordentlich wichtig. Verunreinigungen durch Proteine treten in allen Bereichen von Mensch und Tier auf, z.B. als Fragmente von Haaren, Hautschuppen, Schweiss, Urin, u.s.w. Insbesondere Viren, Bakterien und Pilze bestehen aus Proteinen, was Insbesondere bei erkrankter Haut eine zentrale Rolle spielt wie z.B. Propionibacterium acnes (Akne), Streptococcen (Zahnbelag), Malassezia furfur (Haarschuppen). Ein weiterer Aspekt hygienischer Körperreinigung ist die Entfernung der häufig pathogenen Stoffwechselprodukten. Weiterhin können Proteine Mikroorganismen als Schutz dienen und somit die Wirksamkeit von Köperreinigungsmitteln herabsetzen. Eine effektive Proteinschmutzentfernung ist somit besonders wichtig für die persönliche Hygiene. In der Mundhygiene ist es wichtig, die Bildung von Zahnbelag- eine frühe Matrixbildung aus Kohlenhydraten und Eiweiss - frühzeitig zu unterbinden.

Die Entfernung von partikulärem Schmutz ist auch in der Gesichtspflege und Rasur wichtig. Insbesondere das Gesicht ist Umwelteinflüssen und -verschmutzung ausgesetzt. So können sich z.B. Pollen, sowie Russ- und Staubteilchen an Haut und Haaren adsorbieren. Weiterhin ist die Reinigung von partikulären Teilchen wichtig bei der Haarpflege um Reste von Styling-Produkten leicht aus dem Haar auszuwaschen. Ein Ziel der Körperhygiene stellt daher die effektive Entfernung von Proteinen und partikulären Teilchen dar, egal ob in der Mundhygiene, der Haut oder den Haaren, oder der Intimreinigung.

Weiterhin ist ein gutes Dispergiervermögen für ein Reinigungs- und Pflegemittel wichtig. Unter dem Dispergiervermögen versteht man die Fähigkeit einer oberflächenaktiven Substanz oder eines Mittels, Partikel in die Reinigungslösung zu überführen, also das Schmutztragevermögen. Häufig werden nichtionische Tenside als Dispergiermittel eingesetzt, wie beispielsweise Alkylglucoside, die jedoch wieder haut- und augenreizend sind. Üblicherweise werden für die Entfernung von hartnäckigem Schmutz Tensidgemische eingesetzt. Häufig enthalten Reinigungsprodukte zur mechanischen Reinigung Reibekörper wie Holzmehl, Cellulose, Plastikpartikel, Kaolin, Sand und andere. Besonders hartnäckige Verschmutzungen werden unter Zusatz agressiver organischer Lösungsmittel entfernt, wie z.B. aliphatische Kohlenwasserstoffe. Je häufiger die Produkte angewendet werden, z.B. bei empfindlichen Partien wie Augenbereich, Intimbereich oder auch im gewerblichen Bereich, desto deutlicher werden die nachteiligen Wirkungen der enthaltenen Inhaltsstoffe. So ist zum Beispiel eine Vielzahl der üblicherweise verwendeten Tenside als hautirritierend bekannt. Insbesondere führen auch die üblicherweise verwendeten Lösungsmittel zu einer Entfettung und Austrocknung der Haut durch die Zerstörung des Hydro-Lipid-Mantels. Die Haut wird damit permeabler für toxische oder allergene Stoffe und reagiert durch Hautreizungen und allergene Hautreaktionen.

Zur Vermeidung der vorgenannten Hautproblematik sind in der Patentliteratur eine Vielzahl von Formulierungen vorgeschlagen worden. Nichtsdestoweniger geht die verbesserte Hautverträglichkeit sowie der verbesserte Schutz vor Hautaustrocknung mit einem Verlust an Reinigungswirkung einher. Neben dem ehrgeizigen Ziel eine bessere Hautverträglichkeit bei hoher Reinigungskraft zu erreichen, sollen gleichzeitig die Inhaltsstoffe aus nachwachsenden Rohstoffen bezogen werden. Hierbei haben sich in den vergangenen Jahren Palmöle, für Wasch- und Reinigungsmittel v.a. Palmkernöl, als pflanzliche Basis für Tenside etabliert.

Jedoch wird die Nachhaltigkeit der Tenside auf Basis Palmöle zunehmend in Frage gestellt, da sie aus tropischen Monokulturen stammen, deren Anbaufläche oftmals durch unkontrollierte Rodung wertvoller tropischer Regenwälder gewonnen wird. Diese Pflanzenöle werden aufgrund ihrer technischen Eigenschaften wie vorteilhafte Schaum-, Wasch- und Reinigungsleistung eingesetzt, die sie dank ihrem hohen Laurinsäuregehalt (C12- Fettsäure, gesättigt) besitzen. Alternativ werden in einem geringeren Mass auch andere Palmöle mit hohem Laurinsäuregehalt wie bspw. Kokosöl oder Babassuöl eingesetzt. Zudem finden seit jeher auch tierische Öle und Fette Anwendung. Aufgrund der ungünstigeren Fettsäurezusammensetzung, aus hygienischen (z.B. TSE-Problematik) und weltanschaulichen Gründen (z.B. vegan-Trend) sind allerdings tierische Rohstoffe aus Konsumentensicht oft nicht erwünscht. Weiterhin wird Pflanzenseife seit Jahrtausenden für Wasch- und Reinigungszwecke eingesetzt, deren Anwendung ist aufgrund der Bildung von Kalkseifen und Bindung an einen alkalischen pH-Wert ebenfalls begrenzt.

Die Herausforderung dieser Erfindung besteht daher darin, auf Erdöl, tierische Fette und Palmöle (Palmöl, Palmkernöl, Kokosöl, Babassuöl) als Fettsäurequelle für die Tenside als Hauptkomponente zu verzichten und in einem grösstmöglichen Mass Tenside aus weniger problematischen Quellen, wie zum Beispiel pflanzlichen Ölen aus europäischem Anbau oder Fermentation einzusetzen. Technisch ist dies ein Problem, da aus verfügbaren Ölen, zum Beispiel aus Mitteleuropa, die gewünschte Laurinsäure nicht in ausreichendem Mass gewonnen werden kann und damit heutzutage übliche Reinigungsmittel weitestgehend auf Erdöl oder Palmölen basieren.

US 2004/0265264 offenbart die Verwendung von Sodium PEG-7 Olivenölcarboxylat in "katalytischen" Mengen zur Reduktion von Hautirritation durch das primäre Tensid Natrium Laurethsulfat. In WO 2013098066 wird Natrium PEG-7 Olivenölcarboxylat in vergleichbar geringen Mengen zusammen mit weiteren Tensiden auf Basis Laurylbasis und Biotensid verwendet für ein Babyreinigungsmittel. Das Ausführungsbeispiel offenbart die sensorisch positive Auswirkung auf die Haut durch die Kombination von Biotensiden mit Ölsäure, die Reinigungsleistung wird nicht erwähnt. CN 102716046 offenbart ein pflegendes Shampoo enthaltend Natrium PEG-7 Olivenölcarboxylat und Natrium Laurethsulfat. DE 10147049 offenbart Tensidgemische aus Natrium Cocoylglutamat, Natrium Myristylethersulfat und Natrium PEG-7 Olivenölcarboxylat, welche selektiv Oberflächenlipide statt Sebumlipide auswaschen und somit Hautrauigkeit vermindern.

Damit ist aber das Problem nicht gelöst, nachhaltige Zubereitungen mit einem reduzierten Gehalt an irritierenden Tensiden und Tenside auf Basis von Pflanzölen mit einem hohen Gehalt an langkettigen, vorwiegend ungesättigten Fettsäuren herzustellen, welche eine hohe Reinigungs- und Pflegeleistung, insbesondere auf Eiweiss und partikulären Schmutz, und gleichzeitig eine hervorragende dermatologische Verträglichkeit und geringe Augenreizung aufweisen und zudem über den gesamten pH-Bereich angewendet werden können.

Die komplexe technische Aufgabe der Erfindung hat darin bestanden, Tensidkombinationen zu identifizieren, die neben einem vorteilhaften toxikologischen Profil ausgezeichnete kosmetische Reinigungseigenschaften ausweisen. Gleichzeitig sollen aus Nachhaltigkeitsbetrachtungen entgegen dem Stand der Technik möglichst wenig Tenside abgeleitet aus den Fettsäuren der Palmöle Kokos-, Palm-, Babassu- oder Palmkernöl oder Erdöl verwendet werden. Dies ist technisch insofern anspruchsvoll, da gebräuchliche Tenside auf Basis Palmkernöl in ihrer Fettsäurezusammensetzung einen hohen Anteil an Laurinsäure (C12) aufweisen, welche dem Tensid vorteilhafte Eigenschaften wie Löslichkeit, Stabilität, Benetzungsfähigkeit, Kompatibilität, u.a. verleihen. Die Herausforderung besteht somit darin, pflegende und reinigende Zubereitungen mit einem hohen Gehalt an Tensiden auf Basis von Pflanzenölen mit überwiegenden Fettsäuren mit Kettenlängen über 18 Kohlenstoffatomen und überwiegend ungesättigt herzustellen.

Weiterhin sollten bevorzugt ein oder mehrere Nachteile der herkömmlichen Tensidmischungen betreffend Toxikologie, Reizpotential für Haut, Reizpotential für Augen, biologische Abbaubarkeit, Wassergefährdung, wie sie beispielsweise in der Gefahrenkennzeichnungspflicht nach CLP zu erkennen sind, beseitigt werden.

Die erfindungsgemässen Zubereitungen sollten vorzugsweise zu einem grösstmöglichen Umfang auf natürlichen Rohstoffen basieren und biologisch gut abbaubar sein.

### Beschreibung der Erfindung

Überraschenderweise wurde gefunden, dass Zusammensetzungen, wie in den Ansprüchen definiert und nachfolgend beschrieben, eine oder mehrere der genannten Aufgaben lösen.

Überraschenderweise und für den Fachmann in keiner Weise vorhersehbar, können in den erfindungsgemässen Mitteln die gleiche oder bessere Reinigungsleistung wie in den Vergleichsmitteln erzielt werden bei gleichzeitiger Minimierung der mittelkettigen Tensiden, insbesondere lauryl bzw. coco, ohne die Gesamtkonzentration an Tensiden erhöhen zu müssen. Dies bedeutet, dass bedenkliche Inhaltsstoffe, wie zum Beispiel stark augenreizende Tenside, in den erfindungsgemässen Mitteln teilweise oder ganz substituiert werden konnten. Die erfindungsgemässe Substitution von Tensiden ist insofern erstaunlich, da Tensidsysteme durch Wechselwirkungen untereinander erst die volle Wirkung entfalten und die substituierten Tensidsysteme altbewährte Systeme darstellen.

Unerwarteterweise zeigte sich zudem, dass die erfindungsgemässen Mittel eine für den Fachmann in keiner Weise vorhersehbare Reinigungswirkung auf spezifische Verschmutzungen zeigt. Dies ermöglicht die Herstellung von umweltfreundlichen Mitteln, selbst für hartnäckige Verunreinigungen wie Proteine oder Teilchenschmutz.

Überraschenderweise wurde festgestellt, dass die erfindungsgemässen Mittel wider Erwarten eine sehr hohe Reinigungsleistung auf Proteinschmutz zeigen.

Weiterhin zeigen die Mittel ein nicht vorhersehbar gutes Schmutztragevermögen vergleichbar mit üblicherweise verwendeten Tensidsystemen für diese Aufgabe. Das hohe Dispergiervermögen der erfindungsgemässen Mittel erlaubt zudem eine Stabilisierung von unlöslichen Inhaltsstoffen in der Zusammensetzung, wie bspw. Abrasiva oder UV-Filter, o.ä.

Ein weiterer Vorteil der Erfindung ist, dass anders als in üblicherweise verwendeten anionischen Tensidsystemen, wie z.B. Sodium laureth sulfate oder Sodium lauryl sulfate, das erfindungsgemässe Mittel unempfindlich gegen hartes Wasser ist und damit kein unangenehmes Hautgefühl in Anwesenheit von Calcium- und Magnesiumionen erfolgt.

Dadurch können überraschenderweise auch nichtionische Tenside mit mittelkettigen Kohlenstoffketten mit hohem Irritationspotential, wie z.B. Decyl glucosid, oder Tenside aus Fermentation (Biotenside), die für hartes Wasser üblicherweise eingesetzt werden, minimiert oder ausgeschlossen werden.

Überraschenderweise wurden Synergien in den erfindungsgemässen Mitteln betreffend des Schmutztragevermögens bei hoher Elektrolytkonzentration oder hartem Wasser festgestellt, was den Pflege- und Reinigungsprodukten eine höhere Stabilität verleiht. Das erfindungsgemässe Mittel wird auch in einer konservierungsmittelfreien Ausführungsform offenbart.

Das Schaumverhalten der erfindungsgemässen Mittel entspricht trotz grundsätzlich längerer Kohlenstoffketten der Fettsäureacylreste der Tenside einem reinen Sodium laureth sulfat- Tensidsystem. Wider Erwarten kann in den erfindungsgemässen Mitteln ein stabiler Schaum erzielt werden, wodurch mittelketttige Alkylamidobetaine, Monoethanolamide und Diethanolamide, welche ein erhebliches Irritationspotential aufweisen, reduziert werden. Eine bevorzugte Ausführungsvariante ist daher frei von Alkylamidobetaine, wie bspw. Cocoamidopropylbetain. Eine weitere bevorzugte Ausführungsvariante ist frei von Mono- und Diethanolamiden, wie Cocamid DEA, Cocamid MEA u.a.

Weiterhin ist Gegenstand dieser Anmeldung die Verwendung der erfindungsgemässen Zubereitungen zur Körperreinigung und - pflege, der Haarwäsche, dem Abschminken, der Zahnpflege, und der Rasur.

In einem weiteren Erfindungsgegenstand richtet sich die Erfindung auf ein Wasch- und Reinigungsverfahren umfassend
a) die Bereitstellung einer Wasch- und Pflegelösung umfassend ein Mittel gemäss des ersten Erfindungsgegenstandes
b) in Kontakt bringen von Haut und Haaren mit der der Wasch- oder Reinigungslösung gemäss (a).

### Definitionen:

Technisch unterscheiden sich die Pflanzenöle aus Ölpalmen, Babassu, Palmkernen, oder Kokosnüssen deutlich in der Fettsäurezusammensetzung von den erfindungsgemässen C-18-Pflanzenölen:
In dieser Erfindung werden folgende Pflanzenöle, - fette, -wachse oder-harze als C-18-Pflanzenöl bezeichnet:
Bevorzugt handelt es sich bei den C-18-Pflanzenölen um natürliche Triglyceride. C-18-Pflanzenöle weisen ein Gemisch an gesättigten und ungesättigten Fettsäuren auf, wobei die Fettsäureverteilung von Fettsäuren mit 18 und mehr Kohlenstoffatomen über 60 Gew.-%, besonders bevorzugt über 72 Gew.-% und ganz besonders bevorzugt von über 77 Gew.-% liegt und wobei der Anteil an ungesättigten Fettsäuren über 55 Gew.-%, vorzugsweise über 65 Gew.-% und besonders bevorzugt über 72 Gew.-% liegt.

Bevorzugt liegt der Anteil an Fettsäuren mit 16 und weniger Kohlenstoffatomen unter 30 Gew.-%, bevorzugt unter 27 Gew.-% und besonders bevorzugt unter 17 Gew.-%.

Bevorzugt enthalten die C-18-Pflanzenöle einen Anteil von < 0.5%, besonders bevorzugt > 0.05% Fettsäuren mit 6 Kohlenstoffatomen.

Bevorzugt enthalten die C-18-Pflanzenöle einen Anteil von < 75 Gew-% Hydroxyfettsäuren, bevorzugt < 25 Gew.-%, besonders bevorzugt < 5 Gew.-%.

Bevorzugt enthalten C-18-Pflanzenöle gesättigte oder ungesättigte Fettsäuren mit 20 und mehr Kohlenstoffatomen, wobei deren Gehalt bis zu 96 Gew.-% betragen kann. Bevorzugt enthalten C-18-Pflanzenöle einen Anteil von gesättigten oder ungesättigten Fettsäuren mit 20 und mehr Kohlenstoffatomen von > 0.01 Gew.-% und besonders bevorzugt > 0.05 Gew.-% und ganz besonders bevorzugt > 0.1 Gew-% und äusserst bevorzugt >= 0.2 Gew.-%.

Bevorzugt enthalten die C-18-Pflanzenöle einen Anteil von weniger als 95 Gew.-% Ölsäure, besonders bevorzugt unter 85 Gew.-% Ölsäure; Gew.-% in Definition C-18-Pflanzenöl jeweils bezogen auf den Gesamtgehalt an Fettsäuren im Pflanzenöl.

Aus folgenden Pflanzen bzw. Pflanzenteilen, wie Samen, Kerne, Früchte, Blätter, Wurzeln und anderen, im folgenden C-18-Pflanzen genannt, können C-18 Pflanzenöle gewonnen werden, welche die technischen Merkmale betreffend Fettsäurezusammensetzungen für die erfindungsgemässen Mittel erfüllen und vorzugsweise aus der Gruppe ausgewählt werden umfassend die Pflanzen: Amarant, Anis, Apfel, Aprikose, Argan, Arnika, Avocado, Baumwolle, Borretsch, Brennessel, Brokkoli, Canola, Chia, Hanf, Haselnuss, Buche, Buchsbaum, Distel, Dinkel, Erdnuss, Erdmandel, Flieder, Gartenkresse, Gerste, Granatapfel, Hafer, Hanf, Haselnuss, Heidelbeere, Holunder, Jasmin, Johannisbeere, Johanniskraut, Jojoba, Kamelie, Kamille, Kümmel, Karotte, Kirsche, Koriander, Königskerze, Krambe, Kreuzblättrige Wolfsmilch, Kreuzblütengewächse, Kürbis, Iberischer Drachenkopf, Lavendel, Leindotter, Leinsamen, Liguster, Lupine, Luzerne, Macademia, Mais, Mandel, Marula, Mirabelle, Melone, Mohn, Mongongo, Nachtkerze, Olive, Ölrettich, Ölrauke, Passionsblume, Pekannuss, Pfirsich, Pflaume, Pistazie, Preiselbeere, Purgiernuss (Jatropha), Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut und Wildrose; sowie deren Kombinationen.

Vorzugsweise ist das Öl ausgewählt aus der Gruppe: Aprikose, Avocado, Baumwolle, Brokkoli, Buche, Distel, Dinkel, Erdmandel, Gerste, Hanf, Haselnuss, Jojoba, Kirsche, Königskerze, Krambe, Kreuzblättrige Wolfsmilch, Kürbis, Iberischer Drachenkopf, Leindotter, Leinsamen, Lupine, Luzerne, Macademia, Mandel, Mais, Mohn, Nachtkerze, Olive, Ölrettich, Ölrauke, Pfirsich, Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube und Weizen, sowie deren Kombinationen.

Ganz besonders bevorzugt ist das Öl ausgewählt aus der Gruppe Aprikose, Distel, Erdmandel, Hanf, Krambe, Iberischer Drachenkopf, Leindotter, Leinsamen, Lupine, Luzerne, Mais, Mandel, Olive, Ölrettich, Pfirsich, Raps, Rübsen, Sesam, Sesamblatt, Sonnenblume, Soja, Weintraube und Weizen, sowie deren Kombinationen.

Der Begriff Öle wird in dieser Erfindung stellvertretend für Fette, Wachse und Harze verwendet.

Unter mittelkettigen Tensiden werden im Rahmen der Erfindung Tenside mit gesättigten Alkyl- oder Acylgruppen mit Kettenlängen zwischen 8-18 Kohlenstoffatomen, oder Gemische aus gesättigten Alkyl- oder Acylgruppen mit 8 bis18 Kohlenstoffatomen und ungesättigten C-18-Alkenyl- oder Acylgruppen verstanden, wie sie aus Kokosöl, Palmkernöl oder Babassuöl erhalten werden.

Soweit nicht explizit anders angegeben, steht im Rahmen der vorliegenden Erfindung Alkyl- und Acyl- für gesättigte und ungesättigte Reste.

Im Rahmen der vorliegenden Erfindung steht - soweit nicht anders angegeben- auf Basis von oder abgeleitet von Pflanzenölen, -fetten oder -wachsen stellvertretend für Derivate aus Fettsäuren nach chemischen Umsetzungen- gereinigt oder als Gemisch - und/oder deren synthetische Reaktionsprodukte, wie beispielsweise Additionsprodukte an die Doppelbindung, Reaktionen an der Fettsäurefunktion, wie z.B. Fettalkohole und deren Ether und/ oder Carboxyether, Amine oder Fettsäureamide, Fettsäureester, sowie Imine. Bevorzugt liegen diese Fettsäurederivate als Mischung gemäss der Fettsäureverteilung im nativen Öl vor oder wie sie bei der Umsetzung von natürlich vorkommenden Pflanzenölen oder Fetten anfallen.

Im Rahmen der vorliegenden Erfindung stehen Fettsäuren bzw. Fettalkohole bzw. Fettsäureacyl- bzw. deren Derivate soweit nicht anders angegeben - stellvertretend für verzweigte oder unverzweigte, lineare oder substituierte, insbesondere hydroxysubstituierte, gesättigte, einfach oder mehrfach ungesättigte Carbonsäuren bzw. Alkohole bzw. deren Derivaten mit vorzugsweise 6 bis 24 Kohlenstoffatomen.

Unter Tensid werden im Zusammenhang dieser Erfindung amphiphile organische Substanzen mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Mizellen, lamellare Strukturen u.a. Im Zusammenhang mit dieser Erfindung werden als Tenside Verbindungen bezeichnet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern. PEGylierte Pflanzenöle sind ethoxylierte Pflanzenöle wie definiert in "Safety Assessment of PEGylated Oils as Used in Cosmetics", International Journal of Toxicology November/December 2014, 33. Im Rahmen dieser Erfindung wird die Terminologie verwendet, welche bei kosmetischen Inhaltsstoffen Anwendung findet, welche die Veretherungs- und Veresterungsprodukte von Glyceriden und Fettsäuren mit Ethylenoxid beschreibt. Im Rahmen der Erfindung sind hier insbesondere Vertreter abgeleitet von C-18-Pflanzen bevorzugt;
PEGylierte Fettsäureglyceride sind Mono-, Di- und/oder Triglyceride, welche mit einer spezifischen Anzahl an Alkylenglycol-Einheiten, meist Ethylenglycoleinheiten modifiziert wurden und Nebenprodukte der Reaktion enthalten können. Im Rahmen dieser Erfindung werden PEGylierte Fettsäureglyceride definiert wie in "Safety Assessment of PEGylated Alkyl Glycerides as Used in Cosmetics", Cosmetic Ingredient Review (CIR) 2014. Zu bemerken ist, dass CIR unter "Alkyl" auch ungesättigte Fettsäuren berücksichtigt. Im Rahmen der Erfindung sind hier insbesondere Vertreter abgeleitet von C-18-Pflanzen bevorzugt;

Im Rahmen dieser Erfindung steht soweit nicht anders vermerkt Biotensid für die Biotensid-Glycolipide aus fermentativer Herstellung.

Im Rahmen dieser Anmeldung wird unter "Schwefeltenside" anionische oder amphotere Tenside mit einem schwefelhaltigen hydrophilen Rest verstanden wie z.B. Alkylsulfate, Alkylethersulfate, (alkoxylierte) Sulfosuccinate, (alkoxylierte) Sulfonate, (alkoxylierte) Isethionate, (alkoxylierte) Taurate, Sulfobetaine und Sultaine. Beispiele für sulfathaltige Tenside stellen Sodium Laureth Sulfate, Sodium Lauryl Sulfate, Ammonium Laureth Sulfate, Ammonium Lauryl Sulfate, Sodium Myreth Sulfate, Sodium Coco Sulfate, Sodium Trideceth Sulfate oder MIPA-Laureth Sulfate dar.

Frei von Schwefeltensiden, Phosphaten, Phosphonaten bedeutet, dass die Formulierung keine nennenswerten Mengen an Schwefeltensiden, Phosphaten, Phosphonaten aufweisen. Insbesondere ist hierunter zu verstehen, dass Schwefeltenside, Phosphate, Phosphonate jeweils in Mengen von kleiner 0.1 Gew.-%, bevorzugt von kleiner 0.01 Gew.-% bezogen auf die Gesamtformulierung, insbesondere keine nachweisbaren Mengen, enthalten sind.

"Mindestens ein" wie hierin verwendet, bezieht sich auf 1 oder mehr, beispielsweise 1, 2, 3, 4, 5, 6, 7, 8, 9 oder mehr.

Unter "Reinigungs- und Pflegemittel" wird im Rahmen der Erfindung eine kosmetische oder medizinische Zubereitung zur Pflege von Haut und Haar, Zähne, Zahnfleisch, Nägel sowie zur Entfernung unerwünschter Verunreinigungen, z.B. Make-up, externen Verschmutzungen, Farbe, Erde, u.a., Stoffwechselprodukten von biologischen Vorgängen, z.B. Schweiss, Hautfett u.a. oder Gerüchen verstanden.

Die Zubereitungen können durch einreiben, zudosieren, sprayen, schäumen und andere Methoden (z.B. salben, auflegen, etc.) direkt oder über ein Hilfsmittel, verdünnt oder konzentriert, auf den zu behandelnden Stellen aufgebracht werden. Das Mittel kann reinigen, die Attraktivität einer Person erhöhen, die Gesundheit von Haut und Haar erhalten, inklusive der Rasur, verschönern und pflegen. In den Begriff eingeschlossen ist die Tierpflege und -reinigung.

Unter "Reinigungsleistung" oder "Waschkraft" wird im Rahmen dieser Erfindung die Entfernung von einer oder mehreren Verschmutzungen, Stoffwechselprodukten oder Gerüchen verstanden.

Die Entfernung kann über eine Aufhellung oder Verringerung der Anschmutzung messtechnisch erfasst, oleofaktorisch oder visuell beurteilt werden.

Bevorzugt umfassen Reinigungs- und Pflegemittel neben dem primären Tensid zusätzlich mindestens 4 weitere Inhaltsstoffe ausgewählt aus den Gruppen: Lösungsmittel; weitere Tenside; Komplexbildner; Viskositätsregulatoren; pH-Stellmittel und Puffersubstanzen; Konditionierungsmittel, physiologisch aktive Wirkstoffe, Bindemittel und Konsistenzgeber, Ölkörper, Lösungsvermittler; Abrasiva; Antioxidantien; Vitamine; UV-Filter; Trübungsmittel; Konservierungsmittel; Duftstoffe; Farbstoffe; anorganische Alkali- oder Erdalkalisalze;.

Der HLB (hydrophile-lipophile balance) Wert ist ein Mass für die Hydrophilie, bzw. Lipophilie eines Stoffes, in der Regel eines nichtionischen Tensids. Der Wert kann theoretisch wie in einschlägiger Literatur beschrieben (z.B. nach der Griffin-Methode) oder experimentell durch den Vergleich des Löslichkeitsverhaltens von Standardzusammensetzungen mit bekanntem HLB gemessen werden.

Stoffe, die auch als Inhaltsstoffe von kosmetischen Mitteln dienen, werden nachfolgend gegebenenfalls gemäss der International Nomenclature Cosmetic Ingredient- (INCI-) Nomenklatur bezeichnet. Die INCI-Bezeichnungen sind dem "International Cosmetic Ingredient Dictionary and Handbook, 13th Edition (2010)" zu entnehmen. Herausgeber: The Personal Care Products Council.

Soweit nicht explizit anders angegeben, beziehen sich die angegeben Mengen in Gewichtsprozent (Gew.-%) auf die Gesamtmasse des Mittels. Dabei beziehen sich die prozentualen Mengenangaben auf Aktivgehalte.

Ein erster Gegenstand der Erfindung richtet sich auf eine Zubereitung als kosmetisches oder medizinisches Rinse-off-Reinigungs- oder Pflegemittel enthaltend mindestens eine Verbindung der Formel (I) und zusätzlich eines oder mehrere Tenside (III)

(I) R'-O(C_{b}H_{2b}O)ₒ-{CH₂-CH[O(C_{b}H_{2b}O)ₚR"]-CH₂O}ᵣ-(C_{b}H_{2b}O)_{q}-R‴

mit **b** einer ganzen Zahl zwischen 2 und 4, **o, p, q** unabhängig voneinander ganzen Zahlen von 0 bis 75, wobei o+p+q mindestens 2 ist, r ist 0 oder 1,
**R', R" und R‴** sind jeweils unabhängig voneinander H, CH₂COOM, oder COR, wobei einer oder zwei, bevorzugt einer der Reste R', R" und R‴ CH₂COOM darstellen: Wenn R' = CH₂COOM dann gilt o≠0, wenn R" = CH₂COOM dann gilt p≠0, wenn R" - CH₂COOM dann gilt q≠0, mit **M** = H, Alkali-, Erdalkali-, Ammonium-, oder lösliche Salze organischer Basen, z.B. organisches Ammoniumkation, Mono-, Di- oder Tri- bzw. Tetraalkylammonium, DEA, MEA, TEA, MIPA, oder TIPA;
und wobei einer oder zwei der Reste R', R" und R‴ Fettsäureacylreste RCO darstellen; mit **R** einer gesättigten, ein- oder mehrfach ungesättigten Kohlenwasserstoffkette mit 5-23 Kohlenstoffatomen; welche aus einem C-18-Pflanzenöl abgeleitet ist;
wobei der Anteil von 18 und mehr Kohlenstoffatomen der Fettsäureacylreste RCO in der Verbindung (I) oder in dem Gemisch von Verbindungen (I) über 60 Gew.-%, bevorzugt über 72 Gew.-% und ganz besonders bevorzugt von über 77 Gew.-% liegt;
und der Anteil der ungesättigten Fettsäureacylresten über 55 Gew.-%, vorzugsweise über 65 Gew.-% und besonders bevorzugt über 72 Gew.-% liegt, jeweils bezogen auf den Gesamtanteil an Fettsäureacylresten RCO der ein oder mehreren Verbindungen (I) im Wasch- und Reinigungsmittel;
und wobei die Verbindung (I) vorzugsweise aus einem Gemisch unterschiedlicher Kettenlängen und Sättigungsgrade des Fettsäurerests RCO wie oben definiert besteht und vorzugsweise abgeleitet ist von einem C-18-Pflanzenöl aus der Gruppe umfassend die Pflanzen wie oben definiert.

Bevorzugt liegt der Fettsäurerest RCO mit einem Anteil von 20 oder mehr Kohlenstoffatomen bevorzugt > 0.01 Gew.-%, besonders bevorzugt > 0.05 Gew.-% und ganz besonders bevorzugt ≥ 0.1 Gew.-% beträgt und äusserst bevorzugt ≥ 0.2 Gew.-%.
Bevorzugt liegt der der Anteil an Fettsäurerest RCO mit Fettsäuren von 16 und weniger Kohlenstoffatomen unter 30 Gew.-%, bevorzugt unter 27 Gew.-% und besonders bevorzugt unter 17 Gew.-%;
bevorzugt liegt der der Anteil an Fettsäurerest RCO mit Fettsäuren von 6 und weniger Kohlenstoffatomen < 0.5%, besonders bevorzugt < 0.05%;
bevorzugt liegt der der Anteil an Hydroxyfettsäurerest < 75 Gew-%, bevorzugt < 25 Gew.-%, besonders bevorzugt < 5 Gew.-%; bevorzugt kann der Anteil an Fettsäurerest RCO mit 20 und mehr Kohlenstoffatomen bis zu 96 Gew.-% betragen kann;
bevorzugt liegt der der Anteil des Ölsäureacylrestes RCO bei weniger als 95 Gew.-%, besonders bevorzugt unter 85 Gew.-%, jeweils bezogen auf den Gesamtanteil an Fettsäureresten RCO des eingesetzten Tensids (I);

Tenside dieser Klasse werden bevorzugt erhalten durch die dem Fachmann bekannte Reaktion von Monochloressigsäure an einer terminalen Hydroxylgruppe eines alkoxylierten Fettsäureesters, eines alkoxylierten Alkyl Glycerids, bevorzugt eines Mono- oder Diglycerids, eines alkoxylierten Polyglycerids, oder eines alkoxylierten C-18-Pflanzenöls, oder deren Gemische und anschliessend mit einer Lauge neutralisiert.

Bevorzugt werden die erhaltenen Produktgemische für b=2 allgemein bezeichnet als (i) oder (ii):

Metall PEG-x Pflanzenöl Carboxylat (i)

Metall PEG-x Pflanzenglycerid Carboxylat oder Metall Pflanzenöl Glycereth-x carboxylat (ii)

Mit Metall = Alkalimetallkation, Erdalkalimetallkation, Ammoniumkation (i.e. Carboxylat) oder H (i.e. Carbonsäure) mit Ethoxylierungsgrad x = 2-75, bevorzugt 2-20, besonders bevorzugt 2-8

Geeignete Vertreter der erfindungsgemäss geeigneten Zusammensetzungen umfassen, sind jedoch nicht beschränkt auf: Natrium PEG-6 Mandelöl Carboxylat, Natrium PEG-8 Mandelöl Carboxylat, Natrium PEG-8 Aprikosenkernöl Carboxylat, Natrium PEG-8 Buxus Chinensis Oil Carboxylat, Natrium PEG-6 Aprikosenkernöl Carboxylat, Natrium PEG-40 Aprikosenkernöl Carboxylat, Natrium PEG-8 Arganöl Carboxylat, Natrium PEG-8 Avocadoöl Carboxylat, Natrium PEG-11 Avocadoöl Carboxylat, Natrium PEG-8 Borretschsamenöl Carboxylat, Natrium PEG-8 Macademia Tenuifolia Öl Carboxylat, Natrium PEG-6 Maisöl Carboxylat, Natrium PEG-8 Maisöl Carboxylat, Natrium PEG-8 Traubenkernöl Carboxylat, Natrium PEG-8 Haselnussöl Carboxylat, Natrium PEG-8 Leinsamenöl Carboxylat, Natrium PEG-6 Olivenöl Carboxylat, Natrium PEG-7 Olivenöl Carboxylat, Kalium PEG-7 Olivenöl Carboxylat, Natrium PEG-8 Olea Europaea Öl Carboxylat, Ammonium PEG-7 Olivenöl Carboxylat, PEG-7 Olivenöl Carbonsäure, Natrium PEG-8 Olivenöl Carboxylat, Natrium PEG-10 Olivenöl Carboxylat, Natrium PEG-8 Oryza Sativa Öl Carboxylat, Natrium PEG-8 Prunus Dulcis Carboxylat, Natrium PEG-8 Persea Gratissma Öl Carboxylat, Natrium PEG-8 Passiflora edulis seed oil Carboxylat, Natrium PEG-6 Erdnussöl Carboxylat, Natrium PEG-45 Crambe Absyssinica Seed oil Carboxylat, Natrium PEG-75 Wiesenschaumkrautöl Carboxylat, Natrium PEG-8 Kürbiskernöl Carboxylat, Natrium PEG-3 Rapssamenöl Carboxylat, Natrium PEG-20 Rapssamenöl Carboxylat, Natrium PEG-8 Diestelöl Carboxylat, Natrium PEG-8 Schinziophyton Rautaneii Kernöl Carboxylat, Natrium PEG-8 Sesamsamenöl Carboxylat, Natrium PEG-8 Senum Indicum Öl Carboxylat Natrium PEG-8 Sojabohnenöl Carboxylat, Natrium PEG-20 Sojabohnenöl Carboxylat, Natrium PEG-36 Sojabohnenöl Carboxylat, Natrium PEG-8 Sonnenblumenöl Carboxylat, Natrium PEG-32 Sonnenblumenöl Carboxylat, Natrium PEG-8 Süssmandelöl Carboxylat, Natrium PEG-8 Wassermelonenkernöl Carboxylat, Natrium PEG-8 Weizenkeimöl Carboxylat, Natrium PEG-8 Zea Mais Öl Carboxylat, Natrium PEG-6 Mandelglycerid Carboxylat, Natrium Mandelöl Glycereth-8 Carboxylat, Carboxylat Natrium PEG-20 Mandelglycerid Carboxylat, Kalium PEG-35 Mandelglycerid Carboxylat, Ammonium PEG-60 Mandelglycerid Carboxylat, Natrium Avocadoöl Glycereth-8 Carboxylat, Natrium PEG-11 Avocadoglycerid Carboxylat, Natrium Arganöl Glycereth-8 Carboxylat, Natrium Mandelöl Glycereth-8 Carboxylat, Natrium PEG-14 Mandelglycerid Carboxylat, Natrium Maisöl Glycereth-8 Carboxylat, Natrium PEG-20 Maisglycerid Carboxylat, Natrium PEG-60 Maisglycerid Carboxylat, Natrium PEG-20 Nachtkerzenglycerid Carboxylat, Natrium PEG-60 Nachtkerzenglycerid Carboxylat, Natrium Traubenkernöl Glycereth-8 Carboxylat, Natrium Cannabis Sativa Kernöl Glycereth-8 Carboxylat, Natrium Jojobaöl Glycereth-8 Carboxylat, Natrium PEG-16 Macademiaglycerid Carboxylat, Natrium PEG-25 Moringaglycerid Carboxylat, Natrium PEG-2 Olivenglycerid Carboxylat, Natrium PEG-6 Olivenglycerid Carboxylat, Natrium PEG-7 Olivenglycerid Carboxylat, Natrium Olivenöl Glycereth-8 Carboxylat, Natrium PEG-10 Olivenglycerid Carboxylat, Natrium PEG-40 Olivenglycerid Carboxylat, Natrium Pfirsichkernöl Glycereth-8 Carboxylat, Natrium PEG-60 Passiflora edulis seed glycerid Carboxylat, Natrium PEG-60 Passiflora Incarnata seed glycerid Carboxylat, Natrium PEG-40 Diestelglycerid Carboxylat, Natrium Sojaöl Glycereth-8 Carboxylat, Natrium PEG-35 Soja glycerid Carboxylat, Natrium PEG-75 Soja glycerid Carboxylat, Natrium PEG-2 Sonnenblumenglycerid Carboxylat, Natrium PEG-7 Sonnenblumenglycerid Carboxylat, Natrium Sonnenblumenöl Glycereth-8 Carboxylat, Natrium PEG-10 Sonnenblumenglycerid Carboxylat, Natrium PEG-13 Sonnenblumenglycerid Carboxylat, Natrium PEG-7 Rapsglycerid Carboxylat, Natrium PEG-4 Rapsglycerid Carboxylat, Natrium PEG-10 Canolaglycerid Carboxylat, Natrium PEG-5 Tsubakiateglycerid Carboxylat, Natrium PEG-10 Tsubakiateglycerid Carboxylat, Natrium PEG-20 Tsubakiateglycerid Carboxylat, Natrium PEG-60 Tsubakiateglycerid Carboxylat, Natrium Baumwollöl Glycereth-8 Carboxylat, Natrium Reisöl Glycereth-8 Carboxylat, Natrium Sesamöl Glycereth-8 Carboxylat, Natrium Weizenkeimöl Glycereth-8 Carboxylat.

In dieser Erfindung besonders bevorzugt sind die Vertreter der Tenside mit der allgemeinen Bezeichnung
Metall Pflanzenöl PEG-n Carboxylat,
Mit Metall = Na, K, H, Ammonium; n=ganze Zahlen zwischen 2 und 8,
und den bevorzugten Pflanzenölen ausgewählt aus der Gruppe Distel, Erdmandel, Hanf, Krambe, Iberischer Drachenkopf, Leindotter, Leinsamen, Lupine, Luzerne, Mais, Olive, Ölrettich, Raps, Rübsen, Sesamblatt, Sonnenblume, Soja, Weintraube und Weizen, sowie deren Kombinationen.

Bevorzugt werden die Verbindungen (I), besonders bevorzugt Poly(Oxy-1,2-Ethandiyl), .Alpha.-Carboxy-.Omega.-(Olivenölfettsäuren)Oxy-, Natriumsalz (mit 7 mol EO durchschnittlichem EO-Gehalt), erfindungsgemäss als reinigendes Tensid für Proteinschmutz eingesetzt.

Bevorzugt werden die Verbindungen (I), besonders bevorzugt Poly(Oxy-1,2-Ethandiyl), .Alpha.-Carboxy-.Omega.-(Olivenölfettsäuren)Oxy-, Natriumsalz (mit 7 mol EO durchschnittlichem EO-Gehalt), als reinigendes Tensid für ein verbessertes Schmutztragevermögen eingesetzt.

Bevorzugt werden die Verbindungen (I), besonders bevorzugt Poly(Oxy-1,2-Ethandiyl), .Alpha.-Carboxy-.Omega.-(Olivenölfettsäuren)Oxy-, Natriumsalz (mit 7 mol EO durchschnittlichem EO-Gehalt), als Dispergierungsmittel eingesetzt. Bevorzugt werden die Verbindungen (I), besonders bevorzugt Poly(Oxy-1,2-Ethandiyl), .Alpha.-Carboxy-.Omega.-(Olivenölfettsäuren)Oxy-, Natriumsalz (mit 7 mol EO durchschnittlichem EO-Gehalt), als Stabilisator für partikuläre Inhaltsstoffe eingesetzt.

Bevorzugt werden die Verbindungen (I), besonders bevorzugt Poly(Oxy-1,2-Ethandiyl), .Alpha.-Carboxy-.Omega.-(Olivenölfettsäuren)Oxy-, Natriumsalz (mit 7 mol EO durchschnittlichem EO-Gehalt), als Stabilisator bei hohem Elektrolytgehalt oder hartem Wasser eingesetzt.

Bevorzugt werden die Verbindungen (I), besonders bevorzugt Poly(Oxy-1,2-Ethandiyl), .Alpha.-Carboxy-.Omega.-(Olivenölfettsäuren)Oxy-, Natriumsalz (mit 7 mol EO durchschnittlichem EO-Gehalt), zur Minderung von Augenreizungen durch Tensidsysteme eingesetzt.

Besonders bevorzugt sind Vertreter mit einem HLB > 10.5 und <12.0.

Ganz besonders bevorzugt ist die Zusammensetzung enthaltend die Verbindung (I) mit dem INCI Name: Natrium Olivenöl PEG-7 Carboxylat bzw. Sodium PEG-7 olive oil carboxylate, IUPC-Name Poly(Oxy-1,2-Ethandiyl), .Alpha.-Carboxy-.Omega.-(Olivenölfettsäuren)Oxy-, Natriumsalz mit 7 mol EO durchschnittlichem EO-Gehalt), HLB = 11.

### Verwendung von Fettsäuregemischen

Im Sinne dieser Anmeldung liegt der Verbindung (I) eine Mischung von Fettsäurederivaten auf Basis von C-18-Pflanzenölen mit unterschiedlicher Kettenlänge und Sättigungsgrad zugrunde. Die Mischung folgt bevorzugt der Fettsäureverteilung im nativen Öl oder wie sie bei der Umsetzung von natürlich vorkommenden Pflanzenöle oder Fette anfallen. Indem für die Synthese der Tensidklasse Gemische von Fettsäurederivaten verwendet werden - wie sie bei der Umsetzung von natürlich vorkommenden Pflanzenöle oder Fette anfallen - können die Tenside kostengünstig, ressourceneffizient und umweltschonend produziert werden. Zusätzliche Reinigungsverfahren, wie z.B. die Trennung der Fettsäuren, bzw. Fettsäureester durch fraktionierte Destillation oder zusätzliche Syntheseschritte, wie z.B. zum Fettalkohol, werden hier nicht benötigt. Neben den ökologischen und ökomischen Vorteilen der Verwendung von natürlichen Fettsäuregemischen, zeigen die verwendeten Tensidmischungen eine erhöhte Reinigungsleistung.

Vorzugsweise ist die Polyglykolkette der Verbindung (I) pflanzlichen Ursprungs.

Die Mittel enthalten bevorzugt 0.01 bis 50 Gew.-% eines oder mehrerer Verbindungen (I), bevorzugter 0.25 bis 40 Gew-%, besonders bevorzugt 0.6 bis 20 Gew.-% und ganz besonders bevorzugt 0.6 Gew.-% bis 8 Gew.-%, bezogen auf die Gesamtmasse der Zusammensetzung.

### Weitere Inhaltsstoffe

### Optionale Tenside (II)

Ein weiterer Gegenstand der Erfindung richtet sich auf eine Zusammensetzung umfassend ein oder mehrere zusätzliche Tenside (II), ausgewählt aus den Gruppen der Alkylethersulfate, Alkylsulfate, Acylglutamate, Acylsarcosinate, Sulfosuccinatester, bevorzugt ethoxylierte oder nicht ethoxyliert, Alkylglucoside, Amidoalkylbetaine, Alkanolamide und Amphoacetate
wobei die Tenside gesättigte Alkyl- oder Acylgruppen mit Kettenlängen zwischen 8-18 Kohlenstoffatomen, oder Gemische aus gesättigten Alkyl- oder Acylgruppen mit 8 bis18 Kohlenstoffatomen und ungesättigten C-18-Alkenyl- oder Acylgruppen enthalten, wie sie aus Kokosöl, Palmkernöl oder Babassuöl erhalten werden;
wobei das Verhältnis von (I) zu der Summe der Tenside (II) ≥ 100:1 ist.

Bevorzugte Verbindungen aus der Gruppe der Alkylethersulfate sind:
Alkylethersulfate: Sodium laureth sulfate, Sodium coceth sulfate; Sodium myristyl ether sulfate, Sodium trideceth sulfate; Bevorzugte Verbindungen aus der Gruppe der Alkylsulfate sind: Sodium Lauryl sulfate, Sodium coco sulfate, ammonium lauryl sulfate; Bevorzugte Verbindungen aus der Gruppe der Acylglutamate sind: Sodium cocoyl glutamate, TEA cocoyl glutamate, sodium lauroyl glutamate; Bevorzugte Verbindungen aus der Gruppe der Acylsarcosinate sind Sodium lauroyl sarcosinate, Sodium myristoyl sarcosinate; Bevorzugte Verbindungen aus der Gruppe der Sulfosuccinatester sind bevorzugt Disodium laurylsulfosuccinate, Disodium Laureth sulfosuccinat; Bevorzugte Verbindungen aus der Gruppe der Alkylglucoside, sind Coco glucoside, Lauryl glucoside, Decyl glucoside, C10-C16 alkyl glucoside; Bevorzugte Verbindungen aus der Gruppe der Amidoalkylbetaine und Betaine sind cocoamidopropylbetain und coco betaine; Bevorzugte Verbindungen aus der Gruppe der Alkanolamide sind Lauramide DEA oder MEA, Cocamid DEA oder MEA; Bevorzugte Verbindungen aus der Gruppe der Amphoacetate sind Sodium Coco Amphoacetate, Sodium lauroamphoacetat; wobei Sodium exemplarisch steht für alle Alkali-, Erdalkali-, Ammonium-, oder organisches Ammoniumkationen.

Hierbei gilt insbesondere:
Bevorzugt ist das Verhältnis von (I) zu den Alkylethersulfate ≥ 3,5 : 1 ist, bevorzugt ≥ 5 : 1 und besonders bevorzugt ≥ 10 : 1
Bevorzugt ist das Verhältnis von (I) zu den Alkylsulfate ≥ 3,5 : 1 ist, bevorzugt ≥ 5 : 1 und besonders bevorzugt ≥ 10 : 1.
Bevorzugt ist das Verhältnis von (I) zu den Acylglutamate ≥ 1,5 : 1 ist, bevorzugt ≥ 3,5 : 1 und besonders bevorzugt ≥ 7 : 1.
Bevorzugt ist das Verhältnis von (I) zu den Acylsarcosinate ≥ 1 ,5 : 1 ist, bevorzugt ≥ 3 : 1 und besonders bevorzugt ≥ 7 : 1.
Bevorzugt ist das Verhältnis von (I) zu den Sulfosuccinatester ≥ 1,5 : 1 ist, bevorzugt ≥ 2,5 : 1 und besonders bevorzugt ≥ 5 : 1.
Bevorzugt ist das Verhältnis von (I) zu den Alkylglucoside ≥ 3,5 : 1 ist, bevorzugt ≥ 5 : 1 und besonders bevorzugt ≥ 10 : 1.
Bevorzugt ist das Verhältnis von (I) zu den Amidoalkylbetaine ≥ 1 : 1 ist, bevorzugt ≥ 3 : 1 und besonders bevorzugt ≥ 6 : 1.
Bevorzugt ist das Verhältnis von (I) zu den Alkanolamide ≥ 1 : 1 ist, bevorzugt ≥ 3 : 1 und besonders bevorzugt ≥ 6 : 1.
Bevorzugt ist das Verhältnis von (I) zu den Amphoacetate ≥ 3,5 : 1 ist, bevorzugt ≥ 5 : 1 und besonders bevorzugt ≥ 10 : 1 .

In den erfindungsgemässen Zusammensetzungen ist es gelungen, den Gehalt an mittelkettigen Tensiden bei gleichbleibender oder verbesserter Leistung zu reduzieren. In den erfindungsgemässen Mischungen ist es gelungen, die Einstufung für die Augenreizung gegenüber mittelkettigen Tensidmischungen um mindestens eine Stufe zu verbessern.

Die Mittel enthalten bevorzugt eine oder mehrere Verbindungen (II) bis 1 Gew.-%, bezogen auf die Gesamtmasse der Zusammensetzung.

Die erfindungsgemässen Zusammensetzungen zeichnen sich durch eine hohe Dispergierwirkung aus. In einer bevorzugten Ausführungsform wird auf Fermentationsprodukte verzichtet. Eine besonders bevorzugte Variante enthält keine Biotenside.

Eine bevorzugte Ausführungsvariante enthält keine Tenside der Gruppen (II). Eine bevorzugte Ausführungsvariante enthält ein oder mehrere Tenside der Verbindung (I) sowie weitere Tenside (III) abgeleitet von C18-Pflanzenölen.

### Tenside (III)

Der Gegenstand der Erfindung richtet sich auf Zusammensetzung zusätzlich umfassend ein oder mehrere Tenside (III) ausgewählt aus der Gruppe der Seifen (Alkalimetall- oder Ammonium-Fettsäurecarboxylate), der ethoxylierten Fettsäurealkylester, Fettsäureamide, Fettsäureimine, wobei die Fettsäurereste von einem C-18-Pflanzenöl abgeleitet sind.

Hierbei ist vorzugsweise zumindest eine Seife ausgewählt aus Alkalimetall- oder Ammoniumsalze von gesättigten oder ungesättigten Fettsäuren, hergestellt durch Verseifung von C18-Pflanzenölen, wie oben definiert.

Hierbei ist vorzugsweise zumindest einer der ethoxylierten Fettsäurealkylester, abgeleitet von einem C18-Pflanzenöl ausgewählt aus der Gruppe der ethoxylierten Fettsäuremethylester, ethoxylierte Fettsäureethylester, , besonders bevorzugt ausgewählt aus den Fettsäureestern (D)

Hierbei ist vorzugsweise zumindest einer der Fettsäureamide, abgeleitet von einem C18-Pflanzenöl ausgewählt aus der Gruppe der N-Acylaminosäurederivate, N-Acylaspartat, N-Acylglycinat, N-Acylalaninat, N-Acylsarcosinat, N-Acylglutamat, acylierte Polypeptide, N-Acylaminosulfonsäuren, N-Acyltaurid, alkoxylierten Fettsäureamide, Polyhydroxyfettsäureamide, gegebenfalls ethoxyliert oder carboxyliert , Carbonsäureamidethersulfate, Alkanolamin-Carbonsäure-Kondensate, Amidoalkylpyrrolidone, Amidoamine, N-Alkylamidobetaine, Alkylaminopropionsäure, acylierte Diamine und Polyamine sowie deren Salze, besonders bevorzugt sind ein oder mehrere Tenside (III) ausgewählt aus den Gruppen der Fettsäureamiden (A) oder der Polyhydroxyfettsäureamide (B).

Hierbei ist vorzugsweise zumindest einer der Fettsäureimine, abgeleitet von einem C-18-Pflanzenöl ausgewählt aus der Gruppe der Imidazolcarboxylate, Alkyliminopropionsäure, Amphoacetate. Besonders bevorzugt sind ein oder mehrere Tenside (III) ausgewählt aus den Gruppen der Amphoacetate (C).

### (A) Fettsäureamide

Ein weiterer Gegenstand der Erfindung richtet sich auf eine Zusammensetzung umfassend ein oder mehrere Tenside ausgewählt aus der Gruppe der alkoxylierten Fettsäureamide der Formel (A) folgend,

(A) R- CO-NH-(CₘH₂ₘO)ₙ -H

Wobei **m** die ganze Zahlen 2 oder 3 ist, bevorzugt 2, **n** Zahl im Bereich von 2-10, bevorzugt im Bereich 2-8, ganz besonders bevorzugt 2-4, mit **R** = gesättigte, ein- oder mehrfach ungesättigter Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt; und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (A);
und wobei das Tensid (A) vorzugsweise aus einem Gemisch unterschiedlicher Kettenlängen und Sättigungsgrade des Fettsäurerests RCO wie oben definiert besteht und RCO vorzugsweise abgeleitet ist von einem C-18-Pflanzenöl aus der oben offenbarten Gruppe der Pflanzen.

Besonders bevorzugt sind Fettsäureamide der Formel (A) abgeleitet von Distel, Erdmandel, Hanf, Krambe, Iberischer Drachenkopf, Leindotter, Leinsamen, Lupine, Luzerne, Mais, Olive, Ölrettich, Raps, Rübsen, Sesamblatt, Sonnenblume, Soja, Weintraube und Weizen, sowie deren Kombinationen. Besonders bevorzugt sind Fettsäureamide der Formel (C) mit einem HLB > 10.5 und <12.0. Vorzugsweise ist die Polyglykolkette der Verbindung (A) pflanzlichen Ursprungs.

Die Mittel enthalten bevorzugt bis 30 Gew.-% eines oder mehrere Verbindungen (A), bevorzugter bis 10 Gew-%, besonders bevorzugt bis 0,5 -3 Gew.-% und ganz besonders bevorzugt 0,5 Gew.-% bis 2 Gew.-%, bezogen auf die Gesamtmasse der Mittel.

Erfindungsgemäss äusserst bevorzugt ist das ethoxylierte Fettsäureamid auf Basis Rapsöl, IUPAC Name: Amides, rape oil, N-(hydroxyethyl), ethoxylated; INCI Name: PEG-4-Rapssamenamid, oder Rübsamenamid bzw. PEG-4 rapeseed amide. Handelsname: Amidet N von Kao. Im Vergleich zu dem meist verwendeten Tensid Natrium Laureth Sulfate, weist PEG-4-Rapssamenamid ein deutlich geringeres Augenirritationspotential auf und eignet sich daher hervorragend für die erfindungsgemässen Zusammensetzungen.

### (B) Polyhydroxy Fettsäureamide

Ein weiterer Gegenstand der Erfindung richtet sich auf eine Zusammensetzung umfassend ein oder mehrere Tenside ausgewählt aus der Gruppe der Polyhydroxy Fettsäureamide der Formel (B) folgend, 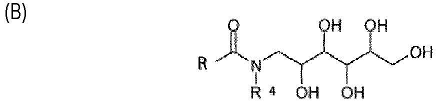
Wobei **R⁴** einen C1-C4-Alkylrest bedeutet, vorzugsweise Methyl und **R** eine gesättigte, ein- oder mehrfach ungesättigter Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt; und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (B);
und wobei das Tensid (B) vorzugsweise aus einem Gemisch unterschiedlicher Kettenlängen und Sättigungsgrade des Fettsäurerests RCO wie oben definiert besteht und wobei RCO vorzugsweise abgeleitet ist von einem C-18-Pflanzenöl aus der oben offenbarten Gruppe der Pflanzen. Erfinderisch besonders bevorzugt sind Fettsäureamide der Formel (B) abgeleitet von Distel, Erdmandel, Hanf, Krambe, Iberischer Drachenkopf, Leindotter, Leinsamen, Lupine, Luzerne, Mais, Olive, Ölrettich, Raps, Rübsen, Sesamblatt, Sonnenblume, Soja, Weintraube und Weizen, sowie deren Kombinationen.

Die Mittel enthalten bevorzugt bis 30 Gew.-% eines oder mehrere Verbindungen (B), bevorzugter bis 10 Gew-%, besonders bevorzugt bis 0,5 -5 Gew.-% und ganz besonders bevorzugt 0,5 Gew.-% bis 3 Gew.-%, bezogen auf die Gesamtmasse der Mittel.

Erfindungsgemäss äusserst bevorzugt ist das Polyhdroxy Fettsäureamid oder Glucamid gemäss Formel (B) auf Basis Sonnenblumenöl, INCI Name: Sunfloweroyl Methylglucamide.

Im Vergleich zu dem meist verwendeten Tensid Natrium Laureth Sulfate, weist Sunfloweroyl Methylglucamide ein deutlich geringeres Augenirritationspotential auf und eignet sich daher hervorragend für die erfindungsgemässen Zusammensetzungen.

### (C) Amphoacetate

Ein weiterer Gegenstand der Erfindung richtet sich auf eine Zusammensetzung umfassend ein oder mehrere Tenside ausgewählt aus der Gruppe der Amphoacetate (C) oder Amphoglycinate, wobei das Tensid (C) vorzugsweise aus einem Gemisch unterschiedlicher Kettenlängen und Sättigungsgrade des Fettsäurerests RCO wie oben definiert besteht und wobei RCO vorzugsweise abgeleitet ist von einem C-18-Pflanzenöl aus der oben offenbarten bevorzugten Gruppe der Pflanzen.

Geeignete Amphoacetate umfassen, sind jedoch nicht beschränkt, auf sodium oliveamphoacetate, sodium sunflowerseedamphoacetate, sodium grapeseedamphoacetate, sodium cottonseedamphoacetate, sodium ricebranamphoacetate, sodium sesamamphoacetate, sodium sweetalmondamphoacetate, sodium peanutamphoacetate, sodium wheat germamphoacetate, und deren Mischungen.

Erfinderisch besonders bevorzugt sind weiterhin Amphoacetate (C) abgeleitet von Distel, Erdmandel, Hanf, Krambe, Iberischer Drachenkopf, Leindotter, Leinsamen, Lupine, Luzerne, Mais, Olive, Ölrettich, Raps, Rübsen, Sesamblatt, Sonnenblume, Soja, Weintraube und Weizen, sowie deren Kombinationen.

Die Mittel enthalten bevorzugt bis 40 Gew.-% eines oder mehrere Verbindungen (C), bevorzugter bis 0,5 - 25 Gew-%, besonders bevorzugt bis 0,5 -10 Gew.-% und ganz besonders bevorzugt 0,5 Gew.-% bis 5 Gew.-%, bezogen auf die Gesamtmasse der Mittel.

Besonders bevorzugt werden Amphoacetate (C) abgeleitet von C-18-Pflanzenölen in einer Konzentration ≥ 5 Gew.-%, bevorzugt ≥ 10 Gew.-%, besonders bevorzugt ≥ 20 Gew.-%, bezogen auf die Gesamtmasse der Tenside in der Zusammensetzung eingesetzt.

In einer bevorzugten Ausführungsform, werden ausschliesslich Amphoacetate (C) abgeleitet von C-18-Pflanzenölen als amphotere Tenside in der Wasch- und Reinigungszusammensetzung eingesetzt.

### (D) Fettsäureester

Ein weiterer Gegenstand der Erfindung richtet sich auf eine Zusammensetzung umfassend ein oder mehr zusätzliche Tenside ausgewählt aus der Gruppe der alkoxylierten Fettsäureester (D), wobei (D) vorzugsweise aus einem Gemisch unterschiedlicher Kettenlängen und Sättigungsgrade des Fettsäurerests RCO wie oben definiert besteht und wobei RCO vorzugsweise abgeleitet ist von einem C-18-Pflanzenöl aus der oben offenbarten bevorzugten Gruppe der Pflanzen.

Geeignete Fettsäureester umfassen, sind jedoch nicht beschränkt auf: Pflanzenöl-PEG- ester (i): PEG-6 Mandelöl, PEG-8 Mandelöl, PEG-8 Aprikosenkernöl, PEG-6 Aprikosenkernöl, PEG-40 Aprikosenkernöl, PEG-8 Avocadoöl, PEG-11 Avocadoöl, PEG-8 Borretschsamenöl, PEG-8 Macademia Tenuifolia Öl, PEG-6 Maisöl, PEG-8 Maisöl, PEG-8 Traubenkernöl, PEG-8 Haselnussöl, PEG-8 Leinsamenöl, PEG-6 Olivenöl, PEG-7 Olivenöl, PEG-7 Olivenöl, PEG-8 Olea Europaea Öl, PEG-7 Olivenöl, PEG-7 Olivenöl, PEG-8 Olivenöl, PEG-10 Olivenöl, PEG-8 Oryza Sativa Öl, PEG-8 Prunus Dulcis, PEG-8 Persea Gratissma Öl, PEG-8 Passiflora edulis seed oil, PEG-6 Erdnussöl, PEG-45 Crambe Absyssinica Seed oil, PEG-75 Wiesenschaumkrautöl, PEG-8 Kürbiskernöl, PEG-3 Rapssamenöl, PEG-20 Rapssamenöl, PEG-8 Diestelöl, PEG-8 Schinziophyton Rautaneii Kernöl, PEG-8 Sesamsamenöl, PEG-8 Senum Indicum Öl, PEG-8 Sojabohnenöl, PEG-20 Sojabohnenöl, PEG-36 Sojabohnenöl, PEG-8 Sonnenblumenöl, PEG-32 Sonnenblumenöl, PEG-8 Süssmandelöl, PEG-8 Wassermelonenkernöl, PEG-8 Weizenkeimöl, PEG-8 Zea Mais Öl; sowie PEG-Pflanzenölglyceridester (ii): PEG-6 Mandelglycerid, Mandelöl Glycereth-8 Ester, PEG-20 Mandelglycerid, PEG-35 Mandelglycerid, PEG-60 Mandelglycerid, Avocadoöl Glycereth-8 Ester, PEG-11 Avocadoglycerid, Arganöl Glycereth-8 Ester, Mandelöl Glycereth-8 Ester, PEG-14 Mandelglycerid, Maisöl Glycereth-8 Ester, PEG-20 Maisglycerid, PEG-60 Maisglycerid, PEG-20 Nachtkerzenglycerid, PEG-60 Nachtkerzenglycerid, Traubenkernöl Glycereth-8 Ester, Cannabis Sativa Kernöl Glycereth-8 Ester, Jojobaöl Glycereth-8 Ester, PEG-16 Macademiaglycerid, PEG-25 Moringaglycerid, PEG-2 Olivenglycerid, PEG-6 Olivenglycerid, PEG-7 Olivenglycerid, Olivenöl Glycereth-8 Ester, PEG-10 Olivenglycerid, PEG-40 Olivenglycerid, Pfirsichkernöl Glycereth-8 Ester, PEG-40 Distelglycerid, Sojaöl Glycereth-8 Ester, PEG-35 Soja glycerid, PEG-75 Soja glycerid Ester, PEG-2 Sonnenblumenglycerid, PEG-7 Sonnenblumenglycerid, Sonnenblumenöl Glycereth-8 Ester, PEG-10 Sonnenblumenglycerid, PEG-13 Sonnenblumenglycerid, PEG-7 Rapsglycerid, PEG-4 Rapsglycerid, PEG-10 Canolaglycerid, Baumwollöl Glycereth-8 Ester, Reisöl Glycereth-8 Ester, Sesamöl Glycereth-8 Ester, Weizenkeimöl Glycereth-8 Ester; sowie ethoxylierte Fettsäurealkylester (iii): Ethoxylierte Rapsmethylester (EO 7-15), Ethoxylierte Rapsethylester (EO 7-15), Ethoxylierte Sojamethylester (EO 7-15), Ethoxylierte Sojaethylester (EO 7-15). Vorzugsweise ist die Polyglykolkette der Verbindung (D) pflanzlichen Ursprungs.

Die Mittel enthalten bevorzugt bis 30 Gew.-% eines oder mehrere Verbindungen (D), bevorzugter bis 20 Gew-%, besonders bevorzugt bis 10 Gew.-% und ganz besonders bevorzugt 1 Gew.-% bis 3 Gew.-%, bezogen auf die Gesamtmasse der Mittel.

Bevorzugt umfasst die Zusammensetzung ein oder mehrere Tenside ausgewählt aus der Gruppe der alkoxylierte Fettsäurester, wobei besonders bevorzugt das Gewichts-Verhältnis von (I) zu der Summe der C18-Pflanzenöl-PEG- ester (i), insbesondere bevorzugt zu PEG-7-Olivenölester ≥ 3,5 : 1 und bevorzugt ≥ 5: 1, besonders bevorzugt ≥ 9 : 1.

Die Mittel enthalten bevorzugt bis 5 Gew.-% eines oder mehrerer C18-Pflanzenöl-PEG-ester (i), bevorzugter bis 3 Gew-%, besonders bevorzugt bis 1 Gew.-%. In einer bevorzugten Ausführungsform der Zusammensetzungen wird kein zusätzlicher C-18-Pflanzenöl-PEG-ester (i) zu der Verbindung (I) zugegeben.

Die Mittel enthalten bevorzugt bis 50 Gew.-% eines oder mehrere Verbindungen (III), bevorzugter bis 40 Gew-%, besonders bevorzugt 0.6 bis 20 Gew.-% und ganz besonders bevorzugt 0.6 Gew.-% bis 8 Gew.-%, bezogen auf die Gesamtmasse der Mittel.

### Weitere optionale Tenside (IV) & Emulgatoren

Unter Tensid werden in diesem Zusammenhang amphiphile organische Substanzen mit grenzflächenaktiven Eigenschaften verstanden, die sich an die Grenzfläche zwischen zwei Flüssigkeiten, wie beispielsweise Öl und Wasser adsorbieren und die Fähigkeit besitzen, die Oberflächenspannung von Wasser zu verringern. In Lösung tendieren Tenside zur Selbstaggregation und bilden Strukturen wie bspw. Micellen, lamellare Strukturen u.a. Im Zusammenhang mit dieser Erfindung werden als Tenside Verbindungen bezeichnet, die die Fähigkeit besitzen, die Oberflächenspannung von Wasser bei 20°C und bei einer Konzentration von 0.5 Gew.-% bezogen auf die Gesamtmenge der Zubereitung auf unter 45 mN/m zu verringern.

Für weitere optionale Tenside, welche vom Fachmann frei mit dem erfindungsgemässen Mittel kombiniert werden können, wird auf die einschlägige Fachliteratur wie z.B. Richard J. Farn, Chemistry and Technology of Surfactants, Blackwell Publishing, verwiesen. Die Tenside umfassen Kohlenwasserstoffketten, abgeleitet aus Fettsäuren oder synthetischen Kohlenwasserstoffen gesättigt oder ungesättigt, substituiert oder nicht-substituiert, linear oder verzweigt mit 4-24 Kohlenstoffatomen in der Kohlenwasserstoffkette umfassen. Optionale Tenside umfassen bevorzugt weitere Tenside aus C-18-Pflanzenölen, sind jedoch nicht auf diese beschränkt.

Weiterhin können in dem erfindungsgemässen Mittel auch zusätzliche Verbindungen enthalten sein, die in der Literatur z.T. unter Emulgatoren geführt werden. Erfindungsgemäss geeignete Emulgatoren sind dem Fachmann allgemein bekannt und können aus der einschlägigen Literatur, beispielsweise Kirk-Othmer, "Encylcopedia of Chemical Technology", 5. Aufl. 2007 entnommen werden.

Im Folgenden werden einige bevorzugte Beispiele genannt:
Bevorzugte Emulgatoren sind lineare gesättigte Alkanole mit 12 - 30 Kohlenstoffatomen, insbesondere mit 16 - 22 Kohlenstoffatomen. Typische Beispiele sind Capronalkohol, Caprylalkohol, 2-Ethylhexylalkohol, Caprinalkohol, Laurylalkohol, Isotride- cylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachidyl- alkohol, Gadoleylalkohol, Behenylalkohol, Erucylalkohol und Brassidylalkohol sowie deren Gemische dieser Alkohole, wie sie bei der technischen Hydrierung von pflanzlichen und tierischen Fettsäuren erhältlich sind.

Erfindungsgemäss besonders bevorzugt sind Fettalkohole abgeleitet aus C-18-Pflanzen, insbesondere Mischungen aus Fettalkoholen welche der natürlichen Zusammensetzung des Pflanzenöls entsprechen oder der Mischung, welche durch Umsetzung des Pflanzenöls zum Fettsäure- bzw. Fettsäureestergemisches mit anschliessender Reduktion zum Fettalkoholgemisch erhalten wird.

Überraschenderweise zeigen die erfindungsgemässen Mittel eine vergleichbare Reinigungsleistung zu üblichen Mitteln mit Schwefeltensiden, auch ohne deren Verwendung. Aus Umweltschutzgründen und den zunehmenden Sulfatgehalten in Trinkwasser ist eine bevorzugte Ausführungsvariante frei von allen Schwefeltensiden.

Weiterhin kann das Mittel optional kationische Tenside enthalten, beispielsweise primäre, sekundäre, tertiäre oder quartäre Alkylammoniumsalze der Formel (RI)(RII)(RIII)(RIV)N+X-, in der RI bis RVI unabhängig voneinander gleich- oder verschiedenartige Alkylreste, verzweigt und unverzweigt, gesättigt oder ungesättigt, unsubstituiert, einfach oder mehrfach substituiert, oder H, wobei X- für ein Anion steht.

Vorzugsweise werden Tenside verwendet, deren hydrophiler Teil aus pflanzlichem Ursprung stammt, ganz besonders bevorzugt abgeleitet von Pflanzen aus Mitteleuropa, wie beispielsweise Zuckertenside oder Aminosäuretenside. Bevorzugt sind auch die Polyglykolreste pflanzlichen Ursprungs.

Die erfindungsgemässen Mittel entwickeln einen stabilen Schaum und machen damit das häufig eingesetzte Cocoamidopropyl Betain entbehrlich. Diese Substanz weist ein erhebliches Irritationspotential auf. Eine bevorzugte Ausführungsvariante ist daher frei von Cocoamidopropyl Betain.

Eine weitere bevorzugte Ausführungsvariante ist frei von Cocamid DEA, Cocamid MEA, welche ebenso zur Schaumstabilisierung Anwendung finden und als hautreizend bekannt sind.

**Zusammensetzung der Tenside im erfinderischen Mittel.** Die Zusammensetzung enthält neben Tensid (I) ein oder mehrere weitere Tenside wie in den Ansprüchen 1 und 2 beschrieben, welche gewählt werden können aus den Gruppen der Tenside (II) und den Gruppen der Tenside (III) sowie den weiteren Tensiden.

Bevorzugt beträgt die Summe der Tenside, abgeleitet von C18-Fettsäuren (I)+(III), bezogen auf die Gesamtmasse der in der Zusammensetzung vorliegenden Tenside > 50 Gew.-%, bevorzugt > 65 Gew.-%, besonders bevorzugt > 75 Gew.-%. In einer besonders bevorzugte Ausführungsvariante beträgt die Summe der Tenside, abgeleitet von C18-Fettsäuren (I)+(III)+ (IV), bezogen auf die Gesamtmasse der in der Zusammensetzung vorliegenden Tenside 100 Gew.-%.

### Weitere Zusatzstoffe & Eigenschaften der kosmetischen Zubereitung

### Chelatbildner

Chelatbildner erhöhen die Stabilität der Zubereitungen, können jedoch auch gegen Zahnstein wirksam sein.

Hierbei ist vorzugsweise zumindest einer der Chelatbildner ausgewählt aus der Gruppe: Aminotrimethylene Phosphonsäure, Beta-Alanine Acetessigsäure, Calcium Disodium EDTA, Chitosan, Zitronensäure und dessen Salze und Hydrate, Cyclodextrin, Cyclohexanediamin Tetraessigsäure, Diammonium Citrat, Diammonium EDTA, Diethylenetriaminepentaacetic Acid, Diethylenetriamine Pentamethylene Phosphorsäure, Dikalium EDTA, Dinatrium Azacycloheptane Diphosphonat, Dinatrium EDTA, Dinatrium Pyrophosphate, EDTA, Ethylenediamine- *N*,*N'*-disuccinic acid (EDDS), Etidronsäure, Galactarsäure, β-Glucan, Gluconsäure, Glucuronsäure, Glucoheptonsäure, HEDTA, Hydroxypropyl Cyclodextrin, Methyl Cyclodextrin, Pentapotassium Triphosphat, Pentasodium Aminotrimethylene Phosphonat, Phosphonobutane tricarbonsäure (PBTC), Pentasodium Ethylenediamine Tetramethylene Phosphonat, Pentasodium Pentetate, Pentasodium Triphosphate, Pentetic Acid, (DTPA), Phytic Acid, Potassium Citrate, Potassium EDTMP, Kalium Gluconate, Kalium Polyphosphate, Kalium Trisphosphonomethylamine Oxide, Ribonsäure, Natrium Chitosan Methylene Phosphonate, Natriumcitrat, Natrium Diethylenetriamine Pentamethylene Phosphonate, Natrium Dihydroxyethylglycinate, Natrium EDTMP, Natrium Glucoheptate, Natrium Gluconate, Natrium Glycereth-1 Polyphosphate, Natrium Hexametaphosphate, Natrium Metaphosphate, Natrium Metasilicate, Natrium Phytate, Natrium Polydimethylglycinophenolsulfonate, Natrium Trimetaphosphate, TEA-EDTA, TEA Polyphosphate, Tetrahydroxyethyl Ethylenediamin, Tetrahydroxypropyl Ethylenediamin, Tetrakalium Etidronat, Tetranatrium Iminodisuccinat (IDS), Tetrakalium Pyrophosphat, Tetranatrium EDTA, Tetranatrium Etidronat, Tetranatrium Pyrophosphat, Trikalium EDTA, Trikalium Dicarboxymethyl Alaninat, Trinatrium EDTA, Trinatrium HEDTA, Trinatrium Methylglycin dieacetic acid (MGDA- Na₃), Trinatrium NTA und Trinatrium Phosphate, Phytinsäure, Plfanzenextrakte wie z.B. Lupinus Albus Seed Extract, Carnosine, Bambusa Arundinacea Leaf Extract, Citrus Paradisi (Grapefruit) Peel Extract, Sambucus Nigra Extract; oder im Falle von Säuren, deren Salze.

Diese Chelatbildner können vom Fachmann frei mit anderen hier genannten Inhaltstoffen kombiniert werden.

In einer bevorzugten Ausführungsform enthalten die erfindungsgemässen Zubereitungen Chelatbildner, die biologisch abbaubar sind. Bevorzugt enthalten die erfindungsgemässen Zubereitungen daher keine Phosphate, keine Phosphonate, kein EDTA und keine Polycarboxylate.

Bevorzugt in dieser Erfindung sind daher folgende Komplexbildner auf Basis erneuerbarer Rohstoffe, bzw. mit einer sehr guten biologischen Abbaubarkeit: Beta-Alanine, Beta-Glucan, Chitosan, Diacetic acid, Cyclodextrin, Ethylenediamine- *N*,*N'*-disuccinic acid (EDDS), Galactarsäure, Gluconsäure, Glucuronsäure, Glucoheptonsäure, Methylcyclodextrin, Hydroxypropyl cyclodextrin, Phytinsäure, Polyasparaginsäure, Natrium Carbonat, Carboxy methyl inulin und Natrium Carboxymethyl inulin (NaCMI), Natrium Dihydroxyethylglycinat, Natrium Iminodisuccinat, Natrium Lignosulfate, Tetranatrium Iminodisuccinat (IDS, Tetranatrium Glutamate Diacetat (GLDA, I-glutamic acid, N,N-di (acetic acid), tetrasodium salt), Trinatrium Methylglycin dieacetic acid (MGDA-Na₃), Zitronensäure, Milchsäure und jeweils deren Salze, Plfanzenextrakte wie z.B. Lupinus Albus Seed Extract, Carnosine, Bambusa Arundinacea Leaf Extract, Citrus Paradisi (Grapefruit) Peel Extract, Sambucus Nigra Extract, oder im Falle von Säuren, deren Salze. Äusserst bevorzugt sind die Chelatbildner Tetranatrium Glutamate Diacetat (GLDA, I-glutamic acid, N,N-di (acetic acid), tetrasodium salt), Tetranatrium Iminodisuccinat (IDS), Ethylenediamine- *N*,*N'*-disuccinic acid (EDDS), Trinatrium Methylglycin dieacetic acid (MGDA- Na₃), Gluconsäure, Glucuronsäure, Glucoheponsäure, Polyasparaginsäure, sowie deren Salze.

Bevorzugte erfindungsgemäße Zubereitungen enthalten mindestens einen Komplexbildner in einer Gesamtmenge von 0,1-20 Gew.-%, vorzugsweise 0,2-15 Gew.-%, insbesondere 0,5-10 Gew.-%, bezogen auf die Gesamtmenge der Zubereitung.

### Abrasiva und Poliermittel

Die erfindungsgemässen Zubereitungen zeigen eine gute Reinigungswirkung, so dass der Zusatz von Abrasiva entbehrlich ist. Hierzu gehören Kunststoffreibemittel auf der Basis von Polyethylen oder Polyurethan, organische Polymere, mineralische Reibemittel wie Kieselsäuren z.B. Gelkieselsäuren, Hydrogelkieselsäuren und Fällungskieselsäuren, Aluminiumhydroxid, Aluminiumoxid, Magnesiumsulfat, Natriumaluminiumsilikate, Calciumcarbonat, Kaolin, Sand, Kreide, Calciumpyrophosphat, Dicalcium-phosphat- dihydrat und andere sowie Reibemittel auf pflanzlicher Basis wie Cellulosederivate, Holzmehl oder Kern- und Schalenmehle, sowie deren Gemische. Insbesondere bevorzugt sind für die Verwendung als Grobreinigung sind Reibemittel auf der Basis von natürlichen Kern- und/oder Schalenmehlen, insbesondere Walnussschalen, Mandelschalen, Hasselnussschalen, Olivenkern-, Aprikosenkern- und Kirschkernmehl oder Perlen aus Wachsen (z.B. Jojobawachse).Für die Verwendung in der Zahnpflege bevorzugt geeignete Poliermittelkomponenten sind calciumarm wie Kieselsäuren, Aluminiumhydroxid, Aluminiumoxid, Natriumaluminiumsilikate, organische Polymere oder Gemische solcher Reibkörper.

Die Konzentration der Reibemittel kann bis zu 50 Gew.-% betragen, bevorzugt 0-30 Gew.-%, bezogen auf die Gesamtmenge der Zubereitung.

### Lösungsmittel

Die erfindungsgemässe Zubereitung kann alle in Kosmetik üblichen Lösungsmittel enthalten. In einer bevorzugten flüssigen oder gelförmigen Ausführungsform enthält die Zubereitung Wasser als Lösungsmittel, wobei mehr als 5 Gew.-%, bevorzugt mehr als 15 Gew.-% und besonders bevorzugt mehr als 25 Gew.-% Wasser enthält, jeweils bezogen auf den Aktivgehalt in der Gesamtmenge der Zubereitung. Besonders bevorzugte Zubereitungen enthalten - bezogen auf ihr Gewicht- 5 bis 99.5 Gew.-%, bevorzugt 15 bis 90 Gew.-%, besonders bevorzugt 25 bis 75 Gew.-% Wasser. Alternativ kann es sich um wasserarme oder wasserfreie Zubereitungen handeln, wobei der Gehalt an Wasser in einer bevorzugten Ausführungsform weniger als 10 Gew.-%, besonders bevorzugt weniger als 8 Gew.-% enthält, jeweils bezogen auf die Gesamtmenge der Zubereitung.

In einer weiteren flüssigen oder gelförmigen Ausführungsform ist die Zubereitung wasserfrei, wobei die Zubereitung ein mildes organisches Lösungsmittel als Hauptlösungsmittel enthält. Dabei ist es bevorzugt, dass die Zubereitung 5 bis 99.5 Gew.-%, bevorzugt 10 bis 90 Gew.-%, besonders bevorzugt 25 bis 75 Gew.-% Lösungsmittel enthält, bezogen auf die Gesamtmenge der Zubereitung.

Hierbei ist vorzugsweise zumindest ein Lösungsmittel ausgewählt aus der Gruppe umfassend Aqua (Wasser), Alcohol (Ethanol), Alkohole, Buteth-3, Butoxydiglycol, Butoxyethanol, Butoxyisopropanol, Butoxypropanol, n-Butyl Alcohol, t-Butyl Alcohol, Butyl-3-hydroxybutyrate, Butylene Glycol, Butyloctanol, C1-C6-Alkane, C7-C15-Alkane, Diethylene Glycol, Diethylenglycol monobutylether, Dimethoxydiglycol, Dimethyl Ether, Dimethyl 2-methylglutarat, Dipropylene Glycol, Dipropylenglycol Phenylether, Ethyllactat, 2-Ethyllactat, Ethyl levulinate glycerol ketal, Ethyl levulinate propylene glycol ketal, Ethyl levulinate ethylene glycol ketal, Ethoxydiglycol, Ethoxyethanol, Ethyl Hexanediol, Fettsäuremethylester z.B. auf Basis C18-Pflanzen, Gammalaverolacton, Glycol, Glycerin, Hexanediol, 1,2,6-Hexanetriol, Hexyl Alcohol, Hexylene Glycol, Isobutoxypropanol, Isopentyldiol, Isopropyl Alcohol (iso-Propanol), Lävulinsäure ester, 3-Methoxybutanol, Methoxydiglycol, Methoxyethanol, Methoxyisopropanol, Methoxymethylbutanol, Methoxy PEG-10, Methylal, Methyl Alcohol, Methyl-9-dodecenoate, Methyl Hexyl Ether, Methylpropanediol, 2-Methyl THF, Neopentyl Glycol, N,N-Dimethyl-9-decenamide, Polyole, PEG-4, PEG-6, PEG-7, PEG-8, PEG-9, PEG-6 Methyl Ether, Pentylene Glycol, PPG-7, PPG-2-Buteth-3, PPG-2 Butyl Ether, PPG-3 Butyl Ether, PPG-2 Methyl Ether, PPG-3 Methyl Ether, PPG-2 Propyl Ether, 1,2-Propanediol, 1,3-Propandiol, Propyl Alcohol (n-Propanol), Propylene Glycol, Propylene Glycol Butyl Ether, Propylene Glycol Propyl Ether, Terpene, z.B. Limonen, Thymol, u.a. insbesondere natürlichen Ursprungs wie Zitronenöl, Lavendelöl, Thymianöl, u.a., Tetrahydrofurfuryl Alcohol, Trimethylhexanol. Erfindungsgemäss können diese Lösungsmittel in einer dem Fachmann durchaus bekannten Art und Weise frei mit anderen Inhaltsstoffen kombiniert werden.

In einer bevorzugten Ausführungsform, werden Lösungsmittel aus der Gruppe Lösungsmittel, die aus pflanzlichen Rohstoffen gewonnen werden und biologisch abbaubar sind, verwendet. Erfindungsgemäss bevorzugte milde organische Lösungsmittel sind daher ausgewählt aus der Gruppe Ethanol, C1-C6-Alkane, C7-C15-Alkane, Dimethyl 2-methylglutarat, Ethyllactat, 2-Ethyllactat, Ethyl levulinate glycerol ketal, Ethyl levulinate propylene glycol ketal, Ethyl levulinate ethylene glycol ketal, Fettsäuremethylester auf Basis C18-Pflanzen, Gammalaverolacton, Glycerin, 2-Methyl THF, Polyole, Pentylene Glycol, 1,2-Propanediol, 1,3-Propandiol, Pentylenglycol, Fettsäurealkylester von C-18-Pflanzen, insbesondere Rapsmethylester, Sonnenblumenmethylester, Sojamethylester, Terpene, z.B. D-Limonen, Tetrahydrofurfuryl Alcohol.

Ganz besonders bevorzugt ist die Ausführungsform enthaltend Ethanol, Glycerin, 1,2-Propanediol, 1,3-Propandiol, Pentylenglycol, oder einen Fettsäurealkylester von C-18-Pflanzen, insbesondere Rapsmethylester und Sojamethylester als mildes organisches Lösungsmittel.

Das Lösungsmittel kann dabei in der wässrigen, der wasserarmen sowie der wasserfreien Ausführungsform enhalten sein, dabei ist es bevorzugt, dass die Zubereitung 0.2- 99.5 Gew.-% Lösungsmittel, besonders bevorzugt 0.5 -98 Gew.-% enthält, wobei die Konzentration jeweils bezogen ist auf den Aktivgehalt in der gesamten Zubereitung.

### pH- Stellmittel und Puffersubstanzen

Der pH-Wert der erfindungsgemässen Zubereitung kann mittels üblicher pH-Regulatoren eingestellt werden, wobei je nach Anwendung dem Fachmann bekannte, unterschiedliche pH-Bereiche von sauer (pH 1-4) zu neutral (pH 5-7) bis hin zu basisch (pH 8-11) eingestellt werden. pH-Stellmittel und Puffersubstanzen, deren Auswahl dem Fachmann keine Mühe bereitet, sind beispielsweise Carbonsäuren, Mineralsäuren, Ammoniak und Amine, z.B. Natriumbicarbonat, Natriumeitrat, Natriumbenzoat, Zitronensäure, Milchsäure, Glycolsäure, Phosphorsäure oder saure Salze, z.B. NaH₂PO4, Alkali- oder Erdalkalihydroxide oder organische Amine, wie Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin. Auch die Verwendung von Omega-Aminosäuren wie Omega-Aminocapronsäure als Alkalisierungsmittel ist möglich.

Die Zubereitung ist über einen weiten pH-Bereich stabil, so werden unterschiedliche Ausführungsformen ermöglicht, wie zum Beispiel Haarfärbebereich zwischen 5 und 11, Rasur/ Haarentfernung zwischen 3 und 10, Seifen und Gesichtswasser pH 5-9, insbesondere ein hautneutraler pH von 5.5. Vorzugsweise hat die Zubereitung einen pH-Wert zwischen 0-14, besonders bevorzugt zwischen 3 und 11.

### Antioxidantien

Bevorzugt ist zumindest ein Antioxidant ausgewählt aus der Gruppe bestehend aus Aminosäuren (z.B. Glycin, Histidin, Tyrosin, Tryptophan) und deren Derivaten, Imidazole (z.B. Urocaninsäure) und deren Derivaten, Peptide wie D,L-Carnosin, D-Carnosin, L-Carnosin und deren Derivaten (z.B. Anserin), Carotinoide, Carotine und deren Derivate, Chlorogensäure und deren Derivate, Liponsäure und deren Derivate, Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, [gamma]-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z.B. [alpha]-Hydroxyfettsäuren, Palmitinsäure, Phytinsäure, Lactoferrin), [alpha]-Hydroxysäuren (z.B. Citronensäure, Milchsäure, Apfelsäure), Numinsäure, Gallensäure, Gallenextrakte, Bilirubin, Biliverdin, EDTA, EGTA und deren Derivate, ungesättigte Fettsäuren und deren Derivate (z.B. [gamma]-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, [alpha]-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid), Superoxid-Dismutase und die erfindungsgemäss geeigneten Derivate (Salze, Ester, Ether, Zucker, Nukleotide, Nukleoside, Peptide und Lipide) dieser genannten Stoffe.

Erfindungsgemäss bevorzugt sind Antioxidantien, die auf Rohstoffen aus Pflanzen, bevorzugt C-18-Pflanzen beruhen, wie beispielsweise Antioxidantien aus den Gruppen der Aminosäuren, Peptide, Cartinoide, Chelatoren, Pflanzenextrakten und Hydroxysäuren, sowie Mischungen derselben.

Die Menge der Antioxidantien (eine oder mehrere Verbindungen) in den Zubereitungen beträgt vorzugsweise 0.001 bis 30 Gew.- %, besonders bevorzugt 0.05-20 Gew.-%, insbesondere 1-10 Gew.-%, bezogen auf die Gesamtmenge der Zubereitung. Diese Antioxidatien können vom Fachmann mit anderen hier genannten Inhaltstoffen kombiniert werden.

### Konditionierungsmittel

Die erfindungsgemässe Zubereitung kann übliche, dem Fachmann bekannte Konditionierungsmittel enthalten, wie beispielsweise monographiert in dem "International Cosmetic Ingredient Dictionary and Handbook", 14. Auflage, K. Schrader, oder den "Fundamentals and Formulations of Cosmetics", 2. Auflage (Hüthig Verlag, Heidelberg, 1989), S. 782-815.

### Keimhemmende, fungizide und antimikrobielle Wirkstoffe

Optional kann die erfindungsgemässe Zubereitung Wirkstoffe gegen Mikroorganismen wie Pilze oder Bakterien enthalten um Geruch, Schuppenbildung, Karies, Akne u.a. zu unterbinden.

Als keimhemmende oder antimikrobielle Wirkstoffe erfindungsgemäß einsetzbar sind Organohalogenverbindungen sowie - halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Desweiteren sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar. Weitere bevorzugte Wirkstoffe sind ausgewählt aus sogenannten präbiotisch wirksamen Komponenten, worunter erfindungsgemäß solche Komponenten zu verstehen sind, die nur oder zumindest überwiegend die unerwünschten Keime der Hautmikroflora hemmen, nicht aber die erwünschten, das heißt, diejenigen, die zu einer gesunden Hautmikroflora gehören. Dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbeondere Extrakte aus Picea sp., Paullinia sp., Panax sp., Lamium album oder Ribes nigrum sowie Mischungen dieser Substanzen.Weitere bevorzugte Wirkstoffe sind ausgewählt aus den keimhemmend wirkenden Parfümölen bzw. ätherischen Ölen.Weiterhin kann das Mittel Enzyminhibitoren enthalten, die die für die Schweißzersetzung verantwortlichen Enzyme, insbesondere die Arylsulfatase, β-Glucuronidase, Aminoacylase, die esterspaltenden Lipasen und die Lipoxigenase, hemmen, z. B. Trialkylcitronensäureester, insbesondere Triethylcitrat, oder Zinkglycinat.Die Menge der Deodorant-Wirkstoffe in den erfindungsgemäßen Zusammensetzungen beträgt 0,1 -10 Gew.-%, vorzugsweise 0,2 - 7 Gew.-%, insbesondere 0,3 - 5 Gew.-% und außerordentlich bevorzugt 0,4 - 1,0 Gew.-%, bezogen auf die Gesamtmenge der Zubereitung.

### Konservierungsmittel

Die erfindungsgemässe Zubereitung kann alle in Kosmetika üblichen Konservierungsmittel enthalten. Diese haben teilweise auch antimikrobielle Eigenschaften.

Erfindungsgemäss geeignet sind beispielsweise Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff- und Stickstoff-Acetale sowie -Formale, Benzamidine, Isothiazole und deren Derivate wie Isothiazolinone, Phtalimidderivate, Pyridinderivate, oberflächenaktive Verbindungen, Guanidine, antimikrobielle amphoterer Verbindungen, Chinoline, 1 ,2-Dibrom-2,4-dicyanobutan, lodo-2-propynyl-butyl-carbamat, lod, lodophore und Peroxide. Derartige Stoffe können z. B. ausgewählt sein aus p- Hydroxybenzoesäuremethyl-, -ethyl- oder propylester, Natriumsorbat, Natriumbenzoat, Bromchlorophen, Triclosan, Phenyl- Salicylsäureester, Biguanide, z. B. Chlorhexidin und Thymol.

Hierbei ist vorzugsweise zumindest ein Konservierungsmittel ausgewählt aus der Gruppe umfassend Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium- , Kalium- und Magnesiumsalicylat), 4-Hydroxybenzoesäure und deren Ester und Alkalimetallsalze (z. B. Methyl-, Ethyl-, Propyl-, Isopropyl-, Butyl-, Isobutyl-, Isodecyl-, Phenyl-, Phenoxyethyl- und Benzylparaben, Hexamidinparaben und - diparaben, Natrium- und Kaliumparaben, Natrium- und Kaliummethylparaben, Kaliumbutylparaben, Natrium- und Kaliumpropylparaben), Alkohole und deren Salze (z.B. Ethanol, Propanol, Isopropanol, Benzylalkohol, Phenethylalkohol, Phenol, Kaliumphenolat, Phenoxyethanol, Phenoxyisopropanol, o-Phenylphenol), Guajacol und dessen Derivate, Chlorhexidin und dessen Derivate (z. B. Chlorhexidindiacetat, -digluconat, und -dihydrochlorid), Hydantoin und dessen Derivate (z. B. DEDM- und DMDM-Hydantoin, DEDM-Hydantoindilaurat), Harnstoff und Harnstoffderivate (z. B. Diazolidinylharnstoff, Imidazolidinylharnstoff), Ferulasäure und deren Derivate (z. B. Ethylferulat), Sorbinsäure und deren Derivate (z. B. Isopropylsorbat, TEA-Sorbat, Natrium-, Kalium-, und Magnesiumsorbat), Isothiazol- und Oxazolderivate (z. B. Methylisothiazolinon, Methylchloroisothiazolinon, Dimethyloxazolidin), quartäre Ammoniumverbindungen (z. B. Polyquaternium-42, Quaternium-8, Quaternium-14, Quaternium-15), Carbamate (z. B. lodopropynylbutylcarbamat), Formaldehyd und Natriumformat, Glutaraldehyd, Glyoxal, Hexamidin, Dehydracetsäure, 2-Bromo-2-nitropropan-1,3-diol, Isopropylkresol, Methyldibromoglutaronitril, Polyaminopropylbiguanid, Natriumhydroxymethylglycinat, Natriumphenolsulfonat, Triclocarban, Thymol, Triclosan, Zinkpyrithion sowie diverse Peptid-Antibiotika (z. B. Nisin). Erfindungsgemäss bevorzugt ist eine milde Konservierung der Zubereitung auf Basis von Wirkstoffen, ausgewählt aus Ethanol, Benzylalkohol, Dehydroessigsäure und deren Salzen, pflanzlichen organischen Säuren, Glycerin, Zitronensäure, Milchsäure, Benzoesäure, Salicylsäure, Kaliumsorbat. Die hier genannten Inhaltstoffe können vom Fachmann mit anderen kosmetischen Inhaltstoffen kombiniert werden.

Die Menge der Konservierungsmittel in den erfindungsgemäßen Zusammensetzungen beträgt 0.001 -10 Gew.-%, vorzugsweise 0.01 - 5 Gew.-% und insbesondere 0.1 - 3 Gew.-%, bezogen auf die Gesamtmenge der Zubereitung.

In einer bevorzugten Ausführungsvariante ist die Zubereitung konservierungsmittelfrei.

### UV-Filter

Es ist auch möglich und vorteilhaft im Sinne der vorliegenden Erfindung, einen Gehalt an UV-Schutzsubstanzen zu verwenden. Die erfindungsgemässe Zubereitung eignet sich insbesondere, da die partikulären Teilchen in der erfindungsgemässen Kombination der Tenside wie offenbart ausserordentlich gut dispergiert werden. Neben dem Effekt, dass dadurch Pigmente gut von einem Substrat gelöst werden können, hat dies gleichzeitig den Effekt, dass Zubereitungen mit Farbe oder Pigmenten stabilisiert werden. Beispiele für pigmentäre UV-Filter sind Zinc oxid, Titan dioxid, Methylene Bis-Benzotriazolyl Tetramethylbutylphenol, u.a. Vorteilhaft können daher erfindungsgemässe Zubereitungen Substanzen enthalten, die UV-Strahlung im UVA und UVB-Bereich absorbieren, wobei die Gesamtmenge der Filtersubstanzen an der gesamten Zubereitung vorzugsweise zwischen 0.1 Gew.-% bis 30 Gew.-% betragen kann. Bevorzugt sind handelsübliche wasser- oder öllösliche UV-Filter. Üblicherweise werden Kombinationen von UV-Filtern eingesetzt, die vom Fachmann mit anderen hier genannten Inhaltstoffen kombiniert werden können.

### Antischuppenmittel

Das kosmetische Mittel kann Kompontenten enthalten, die gegen Kopfschuppen wirksam sind. Beispiele hierfür sind Zinkpyrithion, Selendisulfid, Piroctone Olamin, Climbazol oder auch Pflanzenextrakte wie Vitis Vinifera Seed Extract, Thymus Serpyllum Extrakt, Rosmarinus officialis Leaf Extrakt, Leuconostoc / Radish Root Ferment Filtrate, Leptospermone/ Isoleptospermone/ Flavesone, Leptospermum Scoparoi, Branch/ Leaf Oil, Fragaria ananassa (Strawberry) Seed oil, Adianthum Capillus Veneris Leaf Extrakt, Brennessel Extrakt, Wacholder Extrakt, Thymian Extrakt und andere.

In den erfindungsmässigen Zubereitungen mit Anti-Schuppen-Wirkstoff werden diese in Konzentrationen bis zu 7 Gew.-%, bevorzugt 0.1- 4 Gew.-%, besonders bevorzugt 0.1-2 Gew.-% eingesetzt, jeweils bezogen auf die Gesamtmenge der Zubereitung.

### Farb-, Duft- und Aromastoffe

Um den ästhetischen bzw. organoleptischen Eindruck der erfindungsgemässen Zubereitungen zu verbessern, können alle in Kosmetika üblichen Duft- und Aromastoffe zugesetzt werden.

Bevorzugte Duftstoffe, deren Auswahl dem Fachmann keinerlei Schwierigkeit bereitet, besitzen eine hohe Lagerstabilität und Unempfindlichkeit gegenüber den übrigen Inhaltsstoffen der kosmetischen Zubereitung. Besonders bevorzugt werden Farbstoffe und Duftstoffe, welche in der Literatur nicht als Allergene beschrieben werden und auf natürlichen Rohstoffen basieren, wie zum Beispiel Extrakte aus Pflanzen. In einer weiteren bevorzugten Ausführungsform der Erfindung, werden keinerlei Duftstoffe zugesetzt, beispielsweise für Anwendungen bei Konsumenten mit Allergien und/oder sensibler Haut. Die erfindungsgemäßen Zubereitungen enthalten in der parfümierten Ausführungsvariante die Duftstoffkomponente/n in Mengen von bis zu 5 Gew.-%, bevorzugt 0.1 - 3 Gew.-%, besonders bevorzugt 0.2 -2 Gew.-%; jeweils bezogen auf die Gesamtmenge der Zubereitung; in der unparfümierten Ausführungsform sind keinerlei Duftsstoffe enthalten.

### Wirkstoffe

### Anti-Karies, Wundheilende und entzündungshemmende Wirkstoffe

Eine weitere bevorzugte Gruppe von Inhaltsstoffen, die in den erfindungsgemäßen Mitteln enthalten sein kann, sind Antikaries-Wirkstoffe. Diese können beispielsweise aus organischen oder anorganischen Fluoriden ausgewählt sein, z. B. aus Natriumfluorid, Kaliumfluorid, Natriummonofluorphosphat, Natriumfluorosilikat, Zinkfluorid und Zinn-(II)-fluorid. Bevorzugt sollte eine Menge von 0.01 - 0.5 Gew.-% Fluor in Form der genannten Verbindungen enthalten sein. Die kosmetische Zubereitung kann weitere Wirkstoffe z. B. gegen Zahnfleischentzündungen, Hautentzündungen, insbesondere bei erkrankter oder beanspruchter Haut wie z.B. während der Rasur enthalten. Derartige Stoffe können z. B. ausgewählt sein aus Acetylsalicylsäurederivate, Allantoin, Aloe Vera, Azulen, Harnstoff, Kamillenextrakten, Tocopherol, Panthenol, Pantothensäure Bisabolol, Propolis, Rhodanid, Retinol, Salbeiextrakten. Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Weiterhin können halogenierte Diphenylether, z.B. 2,4-Dichlor-2Hhydroxydiphenylether, 4,4-Dichlor-2-hydroxydiphenylether, 2,4,4-Tribrom-2-hydroxydiphenylether und 2,4,4-Trichlor-2-hydroxydiphenylether (Triclosan) verwendet werden. Eingesetzt werden können zahlreiche Pflanzenextrakte, wie einige Beispiele stellvertretend für die Klasse der Pflanzenextrakte in den Ausführungsbeispielen offenbart. *Diese Wirkstoffe werden bevorzugt in Mengen von 0.01-* 5 *Gew.-%* in die erfindungsgemäßen Zubereitungen eingesetzt, Gew.-% bezogen auf die Gesamtmenge der Zubereitung.

### Anti-Akne Wirkstoffe

Das Mittel kann weiterhin übliche Wirkstoffe gegen Akne enthalten, wie beispielsweise Azelainsäure, Antibiotika, Retinoide, Zinc Oxid, α-Hydroxysäuren, Linolsäure, pflanzliche Extrakte wie z.B. Kamillenextrakte, Teebaumöl, Grüner Tee und andere.Sie werden bevorzugt in Mengen von 0.01- 5 Gew.-% in die erfindungsgemäßen Zubereitungen eingesetzt, Gew.-% bezogen auf die Gesamtmenge der Zubereitung.

### Weitere Wirkstoffe

Weiterhin können andere Wirkstoffe in dem kosmetischen Mittel enthalten sein, wie beispielsweise Anti-Ageing-Wirkstoffe, Peptide, UV-Absorber, Vitamine & Mineralstoffe (z.B. Ascorbinsäure, Biotin, Tocopherol oder Rutin, Niacin), Mineralsalze wie z.B. Mangan-, Zink- oder Magnesiumsalze, weitere pflanzliche Extrakte.Sie werden bevorzugt in Mengen von 0.01- 5 Gew.-% in die erfindungsgemäßen Zubereitungen eingesetzt, Gew.-% bezogen auf die Gesamtmenge der Zubereitung.

### Haarfarben

Die erfindungsgemässen Zubereitungen können auch Farbstoffe zur direkten oder oxidativen Färbung von Haaren enthalten. Auf Basis der erfindungsgemässen Zubereitungen können sowohl Haarfärbemittel als auch Tönungsshampoos hergestellt werden. Die Farbstoffe sind dem Fachmann durchaus geläufig und können frei mit der erfinderischen Zubereitug kombiniert werden.

### Bindemittel/ Konsistenzgeber

Die erfindungsgemässe Zubereitung kann als Bindemittel bzw. Konsistenzregler dienen z. B. natürliche und/oder synthetische wasserlösliche Polymere wie Alginate, Carrageenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere Polyvinylalkohol, Polyvinylpyrrolidon, Polyethylenglycole. Sie werden bevorzugt in Mengen von 0,01- 10 Gew.-% in die erfindungsgemäßen Zubereitungen eingesetzt, Gew.-% bezogen auf die Gesamtmenge der Zubereitung.

### Viskosität

Die erfindungsgemässe Zubereitung kann alle in der Kosmetik üblichen, dem Fachmann bekannten, Viskositätsregulatoren enthalten vorzugsweise ist zumindest ein Viskositätsregulatoren ausgewählt aus der Gruppe der organische abgewandelte Naturstoffe (Carboxymethylcellulose und andere Celluloseether, Hydroxyethyl- und -propylcellulose und dergleichen, Kernmehlether), organische vollsynthetische Verdickungsmittel (Polyacryl- und Polymethacryl-Verbindungen, Vinylpolymere, Polycarbonsäuren, Polyether, Polyimine, Polyamide) und anorganische Verdickungsmittel (Polykieselsäuren, Schichtsilikate, Tonmineralien wie Montmorillonite, Zeolithe, Kieselsäuren), sowie organische natürliche Verdickungsmittel (Agar-Agar, Carrageen, Xanthan, Traganth, Gummi arabicum, Alginate, Pektine, Polyosen, Guar-Mehl, Johannisbrotbaumkernmehl, Stärke, Dextrine, Gelatine, Casein) und Mischungen derselben. Bevorzugte Viskositätsregulatoren sind natürliche organische Verdickungsmittel aus pflanzlichen Rohstoffen - einschliesslich Algen - beispielsweise Polysaccharide wie Pektine oder Stärke. Des Weiteren bevorzugt sind biotechnologisch hergestellte Verdickungsmittel mithilfe von nicht genmodifizierten Organismen (non GMO), wie beispielsweise Xanthan. Bevorzugt sind zudem mineralische Verdickungsmittel wie Polykieselsäuren, Schichtsilikate, Tonmineralien wie Montmorillonite, Zeolithe, Kieselsäuren. Bevorzugt werden in den kosmetischen Zubereitungen bis zu 5 Gew.-% der Verdickungsmittel eingesetzt, besonders bevorzugt 0.01- 3 Gew.-%, bezogen auf die Gesamtmenge der Zubereitung.

### Schaum

Der erfindungsgemässen Zubereitungen können zusätzliche Schaumbildner zugegeben werden. Eine weitere bevorzugte Ausführungsform der erfindungsgemässen kosmetischen Zubereitung enthält Saponine als Schaumbildner. Geeignet sind beispielsweise Saponine aus der indischen Waschnuss (Sapindus mukorossi), Koreanischen Ginsengs (Panax ginseng), Agavengewächsen, Inka-Gurke (Cyclanthera pedata), Süssholz *(Glycyrrhiza glabra)* und Seifenrinde (*Quillaja saponaria* Molina) und Mischungen derselben. Besonders bevorzugt werden Saponine, die aufgrund ihres Vorkommens keine langen Transportwege erfordern, in der erfindungsgemässen Zubereitung eingesetzt. Hierbei handelt es sich um Saponine aus folgenden bevorzugten Pflanzen: Efeu (Hedera), Schlüsselblume (Primula veris), Vogelmiere (Stellaria media), Wald-Sanickel (Sanicula europaea), Dornige Hauhechel (Ononis spinosa), Hülsenfrüchten (Leguminosae), Spinat (Spinacia), Spargel (Asparagaceae), Hafer (Avena), (Ononis spinosa), Schattenblümchen (Maianthemum bifolium), Seifenkraut (Saponaria officinalis), Walnuss (Aesculus hippocastanum), Acker-Gauchheil (Anagallis arvensis), Gelber Hohlzahn (Galeopsis segetum), Karthäuser-Nelke (Dianthus carthusianorum), Ackerschachtelhalm (Equisetum arvense) und Mischungen derselben. Die Menge an Saponinen beträgt üblicherweise bis zu 5 Gew.-%, vorzugsweise 0.001 bis 3 Gew.-%, insbesondere 0.01 bis 2 Gew.-%, bezogen auf die Gesamtmenge der Zubereitung.

### Ölkörper

Die erfindungsgemässe Zubereitung kann übliche, dem Fachmann bekannte Ölkörper enthalten. Zu Ölkörpern zählen wasserunlösliche, organische Verbindungen, wobei die Wasserunlöslichkeit erfindungsgemäß als eine Löslichkeit von weniger als 10 Gew.-% bei 20° C verstanden wird. Zu den erfindungsgemäß einsetzbaren Ölkörpern zählen beispielsweise Glyceride, Kohlenwasserstoffe, Silikonöle, Dialkylether, Dialkylcarbonate, Wachsester, etc. Auch beliebige Gemische von Ölkörpern sind erfindungsgemäß einsetzbar. Als Fettsäure sind erfindungsgemäß C6-C30-Fettsäuren geeignet. Die Fettsäuren können verzweigt oder unverzweigt, hydroxyliert oder nicht-hydroxyliert, gesättigt oder ungesättigt sein. Erfindungsgemäß bevorzugt ist die Verwendung Glyceride pflanzlicher Herkunft, besonders bevorzugt sind Glyceride der C-18-Pflanzen, z.B. Sojaöl, Baumwollsaatöl, Sonnenblumenöl, Leinöl, Mandelöl, Maisöl, Olivenöl, Rapsöl, Sesamöl, Distelöl, Weizenkeimöl, Pfirsichkernöl und dergleichen. Zu den erfindungsgemäß einsetzbaren Ölkörpern zählen auch natürliche und synthetische, aliphatische und/oder naphthenische Kohlenwasserstoffe, wie z. B. Squalan, Squalen, Paraffinöle, Isohexadecan, Isoeicosan oder Polydecene sowie Dialkycyclohexane. Erfindungsgemäss sind auch Siliconöle geeignet. Siliconöle sind aus Nachhaltigkeitsbetrachtungen in den erfindungsgemässen Zubereitungen nicht bevorzugt. Geeignete und erfindungsgemäß bevorzugte Öle sind weiterhin verzweigte gesättigte oder ungesättigte Fettalkohole mit 6-30 Kohlenstoffatomen. Diese Alkohole werden häufig auch als Guerbet-Alkohole bezeichnet, da sie nach der Guerbet-Reaktion erhältlich sind. Beispiele bevorzugter Alkoholöle sind Hexyldecanol, Octyldodecanol, 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24. Weitere geeignete Ölkomponenten sind Mischungen aus Guerbetalkoholen und Guerbetalkoholestern, z.B. Hexyldecanol und Hexyldecyllaurat. Als Ölkörper kommen auch Ester von linearen, gesättigten oder ungesättigten C6-C30-Fettsäuren mit linearen oder verzweigten, gesättigten oder ungesättigten C6-C30-Fettalkoholen bzw. Ester von verzweigten C6-C30-Carbonsäuren mit linearen oder verzweigten, gesättigten oder ungesättigten C6-C₃₀-Fettalkoholen, welche hydroxyliert sein können (sogenannte Wachsester) in Frage. Unter den Wachsestern seien expemplarisch folgende Vertreter genannt: Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearyl- myristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearyl- behenat, Isostearyloleat, Oleylmyristat, Oleylpalmitat, Oleylstearat, Oleylisostearat, Oleyloleat, Oleylbe- henat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Vorzugsweise werden ein- oder mehrfach ungesättigte Wachsester, wie beispielsweise Oleyloleat und Oleylerucat eingesetzt. Insbesondere bevorzugt sind diese Wachsester abgeleitet aus C18-Pflanzen. Daneben eignen sich auch Ester von linearen C6-C22-Fettsäuren mit verzweigten Alkoholen, insbesondere 2-Ethylhexanol, Ester von C2-C38-Alkylhydroxycarbonsäuren mit linearen oder verzweigten C6-C22-Fettalkoholen, Ester von linearen und/oder verzweigten Fettsäuren mit mehrwertigen Alkoholen (wie z. B. Propylenglycol, Dimerdiol oder Trimertriol) und/oder Guerbetalkoholen, sowie Ester von C6-C22-Fettalkoholen und/oder Guerbet- alkoholen mit aromatischen Carbonsäuren, insbesondere Benzoesäure, Ester von C2-C12-Dicarbonsäuren mit linearen oder verzweigten Alkoholen mit 1 bis 22 Kohlenstoffatomen (z. B. Dioctyl Malate) oder Polyolen mit 2 bis 10 Kohlenstoffatomen und 2 bis 6 Hydroxylgruppen. Weitere beispielhafte Vertreter sind: Hexyldecylstearat, Hexyldecyllaurat, Isodecylneopentanoat, Isononylisononanoat, 2-Ethylhexylpalmitat und 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Ethylenglycoldioleat und -dipalmitat, Linolylmyristat, Linolylpalmitat, Linoleylmyristat, Linoleylpalmitat, Isostearyloleat, Isostearylbehenat, Isostearyloleat, , Linoylstearat, Linoleylstearat, Oleyloleat, Oleyllinolat, Oleyllinolenat, Oleylrizinat, Oleylerucat, Linolyloleat Linolyllinolat, Linolylrizinat, Linolylerucat, Linolenyloleat, Linolenyllinolat, Linolenyllinenolat, Linolenylrizinat, Linolenylerucat, Erucyllinolat, Erucyllinolenat, Erucylrizinat, wobei die Linolenderivate alpha-, sowie gamma-Linolenderivate einschliessen.

Als Ölkörper geeignet sind lineare oder verzweigte, symmetrische oder unsymmetrische, gesättigte oder ungesättigte Di-n-alkyl(en)ether mit 12 bis 24 C-Atomen pro Alkyl(en)gruppe, wie z. B. Di-n-octylether, Di-(2-ethylhexyl)-ether, Laurylmethylether oder Octylbutylether, Didodecylether oder Dibehenylether, wobei bevorzugt Ether abgeleitet aus C18-Pflanzen verwendet werden. Besonders bevorzugt sind als Ölkörper lineare oder verzweigte, gesättigte oder ungesättigte C6-C40-Fettalkoholcarbonate geeignet. Diese Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in Chem.Rev. 96, 951 (1996). Typische Beispiele für Dialkyl(en)carbonate sind vollständige oder partielle Umesterungsprodukte von Dimethyl- und/oder Diethylcarbonat mit Capronalkohol, Caprylalkohol, 2-Ethylhexylalkohol, Caprinalkohol, Laurylalkohol, Isotride- cylalkohol, Myristylalkohol, Cetylalkohol, Palmoleylalkohol, Stearylalkohol, Isostearylalkohol, Oleylalko- hol, Elaidylalkohol, Petroselinylalkohol, Linolylalkohol, Linolenylalkohol, Elaeostearylalkohol, Arachidyl- alkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen anfallen. Bevorzugt geeignet Dialkylcarbonate abgeleitet von C-18-Pflanzenölen. Weitere erfindungsgemäß geeignete Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl- /Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat. Weiterhin geeignet sind Ölkörper aus Ester von linearen oder verzweigten, gesättigten oder ungesättigten Fettalkoholen mit 2-30 Kohlenstoffatomen mit linearen, oder verzweigten gesättigten oder ungesättigten Fettsäuren mit 2-30 Kohlenstoffatomen, die hydroxyliert sein können. Weitere Öle, sind ausgewählt aus den Anlagerungsprodukten von Ethylenoxid und/oder Propylenoxid an ein- oder mehrwertige C₃₋₂₀-Alkanole wie Butanol, Butandiol, Myristylalkohol und Stearylalkohol, z. B. PPG-14-Butylether, PPG-9-Butylether, PPG-10-Butandiol, PPG-3-Myristylether und PPG-15-Stearylether. Vorzugsweise werden Ölkörper eingesetzt, die auf C-18-Pflanzen basieren, wie beispielsweise Pflanzenöle und entsprechende Mono-/Di- /Triglyceridmischungen auf Basis von gesättigten und ungesättigten C6-C24-Fettsäuren, Ester von C6-C24-Fettalkoholen und/oder Fettalkohole mit Carbonsäuren oder Polyolen, wie zum Beispiel Zucker mit 2 bis 10 Kohlenstoffatomen und 2 bis 6 Hydroxylgruppen.

### Öle, Fette und Wachs

Hierbei ist vorzugsweise mindestens ein Öl, Fett oder Wachs ausgewählt aus den Gruppen: Tri-,Di-, und Mono-Glyceride, Wachse, wie z. B. Bienenwachs, Bürzelfett, Candelillawachs, Carnaubawachs, Ceresin, Espartograswachs, Guarumawachs, Japanwachs, Korkwachs, Lanolin (Wollwachs), Mikrowachse, Montanwachs, Ozokerit (Erdwachs), Petrolatum, Paraffinwachse, Ouricourywachs, Reiskeimölwachs, Schellackwachs, Sonnenblumenwachs, Fruchtwachse wie Orangenwachse, Zitronenwachse, Grapefruitwachse, Zuckerrohrwachs, chemisch modifizierte Wachse (Hartwachse), wie z. B. Montanesterwachse, Sasolwachse, hydrierte Jojobawachse, hydriertes Rinzinusöl sowie synthetische Wachse, wie z. B. Polyalkylenwachse und Polyethylenglycolwachse, Lecithine, Phospholipide z.B. Kephaline, Sphingosine bzw. Sphingolipide Desweiteren sind auch Mischungen der genannten Öle und Wachse möglich. Erfinderisch besonders bevorzugt eingesetzt werden Pflanzenöle, bzw. Triglyceride, wie sie aus den Pflanzen der gemässigten Zonen isoliert werden können. Ganz besonders bevorzugt werden Öle der Pflanzen und Pflanzenfamilien wie sie in der Anmeldung im Abschnitt "C18-Pflanzen" beschrieben wurden. Die Ölkörper können in den erfindungsgemässen Zubereitungen in einer Menge von 0.2 bis 80 Gew.-%, bevorzugt Mengen von 0.2 bis 70 Gew.-%, bezogen auf die Gesamtmenge der Zubereitung.

### Perlglanzwachse

Hierbei ist vorzugsweise mindestens ein Perlglanzwachs ausgewählt aus den Gruppen: Alkylenglycolester, speziell Ethylenglycoldistearat; Fettsäurealkanolamide, Partialglyceride, Ester von mehrwertigen, gegebenenfalls hydroxysubstituierte Carbonsäuren mit Fettalkoholen mit 6 bis 24 Kohlenstoffatomen, Fettstoffe, wie beispielsweise Fettalkohole, Fettketone, Fettaldehyde, Fettether und Fettcarbonate, die in Summe mindestens 24 Kohlenstoffatome aufweisen; Fettsäuren wie Stearinsäure, Hydroxystearinsäure oder Behensäure, Ringöffnungsprodukte von Olefinepoxiden mit 12 bis 24 Kohlenstoffatomen mit Fettalkoholen mit 12 bis 24 Kohlenstoffatomen und/oder Polyolen mit 2 bis 15 Kohlenstoffatomen und 2 bis 10 Hydroxylgruppen sowie deren Mischungen. Erfindungsgemäss bevorzugt sind Perlglanzwachse basierend auf C18-Pflanzen. Die Einsatzmenge der Perlglanzwachse kann - bezogen auf die Gesamtmenge der Zubereitung - bei 0.1 bis 5, vorzugsweise 0.5 bis 3 und insbesondere 1 bis 1.5 Gew.-% liegen.

### Weitere Inhaltsstoffe

Neben den bisher genannten Komponenten können die erfindungsgemässen Zubereitungen weitere übliche Inhaltsstoffe für Kosmetika enthalten, die dem Fachmann durchaus bekannt sind. Die erfindungsgemässen kosmetischen und dermatologischen Zubereitungen können dementsprechend ferner kosmetische Hilfsstoffe enthalten, wie sie üblicherweise in solchen Zubereitungen verwendet werden; beispielsweise, desodorierende Mittel, Antitranspirant-Wirkstoffe, Desensibilisierende Zusätze (z.B. Hydroxylapatit, Arginin mit Calciumcarbonat, Zinn-, Kalium-, Strontiumchlorid, Kaliumnitrat u.a.), Konsistenzgeber, kationische Polymere, Filmbildner, Überfettungsmittel, Stabilisatoren, biogene Wirkstoffe, Trübungsmittel, ferner Eiweissderivate wie Gelatine, Collagenhydrolysate, Aminosäuren, Oligo- oder Polypeptide auf natürlicher und synthetischer Basis, DNA- oder RNA-Oligonucleotide, Desoxyzucker oder Desoxyzucker-Bausteine enthaltende Polysaccharide, Kreatin, Xanthin-Derivate, z.B. Coffein, Theophyllin, Theobromin, Aminophyllin, Eigelb, Honig, Lanolin und Lanolinderivate, Stoffe mit keratolytischer und keratoplastischer Wirkung, Enzyme und Trägersubstanzen, Pflanzenextrakte, Vitamine, Provitamine und deren Ester, Antiageing- Mittel, Füllstoffe, Pigmente, , Suspendiermittel, Puffergemische, oberflächenaktive Substanzen, weichmachende, anfeuchtende und/oder feuchthaltende Substanzen, entzündungshemmende Substanzen, Meristem Wirkstoffe, Flavonoide und flavonoid-reiche Pflanzenextrakte, Isoflavonoide und isoflavonoid-reiche Pflanzenextrakte, Ubichinon und ubichinon-reiche Pflanzenextrakte, Silymarin, selbstbräunende Wirkstoffe, haarentfernende Wirkstoffe und haarwachstumanregende bzw. haarwuchsregulierende Wirkstoffe, sebumregulierende Wirkstoffe, hautaufhellende Wirkstoffe oder Weissmacher in Zahnpasten (z.B. Phosphate oder Papain), Lichtschutzmittel, Insektenrepellentien, Bakterizide, Salze, antimikrobiell, proteolytische oder wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte, sowie Gemische derselben. Diese sind vorzugsweise in einer Menge von 0,001 - 20 Gew.-% in der Zubereitung enthalten.

### Verfahren

Ein weiterer Gegenstand der Erfindung richtet sich auf ein Verfahren zur Reinigung und/oder Pflege.

Verfahren zur Reinigung und Pflege zeichnen sich im allgemeinen dadurch aus, dass in mehreren Verfahrensschritten verschiedene aktive Substanzen auf den Körper, bzw.- das Körperteil oder auf ein Substrat, z.B. ein Textil, ein Tuch, eine Bürste etc. aufgebracht und nach der Einwirkzeit abgewaschen werden, oder dass der Körper, der Mund, die Zähne, die Haare, das Fell oder die Haut in sonstiger Weise mit der erfindungsgemässen Zubereitung behandelt wird.

Das Reinigungs- und/oder Pflegeverfahren umfassend die Verfahrensschritte:
a) Bereitstellen einer Zubereitung umfassend ein Reinigungs- oder Pflegemittel gemäss den vorhergehenden Beschreibungen
b) In Kontakt bringen von Körper, Zähne, Zahnfleisch, Haaren oder Fell mit dem Reinigungs- oder Pflegemittel gemäss a) Alle Sachverhalte, Gegenstände und Ausführungsformen, die für die Zubereitung beschrieben sind, sind auch auf das Reinigungs-und Pflegeverfahren sowie deren Verwendung anwendbar und umgekehrt.

### Verwendung

Gegenstand dieser Anmeldung ist die bevorzugte Verwendung der erfindungsgemässen Zubereitungen als oder zur Herstellung von Reinigungs- und Pflegemitteln für Körper, Mund und Zähne, Haut und Haare sowie zur Tierpflege.

Wie oben beschrieben, betrifft die Erfindung insbesondere die Verwendung der Zubereitung zur Entfernung von Protein- oder partikularer Verschmutzung. Bevorzugt ist auch die Verwendung zur Dispergierung von Partikeln in der Zusammensetzung. Die Zubereitung eignet sich daher insbesondere für abrasive Anwendungen.

Durch die erfindungsgemässe Tensidkombination kann die Augenreizung der Tensidkombination abgemildert werden. Daher ist Zubereitung bei einem neutralen pH-Wert zwischen 5,5 und 7,2 besonders bevorzugt für die Verwendung in Schleimhautnähe wie Shampoo, Handseife, Intimwaschlotionen, Augen-Makeup-Entferner, Makeup Entferner, Duschzubereitungen, Kontaktlinsenreinigung und andere.

In einer bevorzugten Ausführungsform wird die Zubereitung in bei saurem pH zwischen 0 und 7, bevorzugt zwischen 1 und 6 eingesetzt und ganz besonders bevorzugt bei einem pH zwischen 2 und 5.5. Überraschenderweise zeigt die erfindungsgemässe Zubereitung eine ausgesprochen gute Eignung im Sauren. Das erfindungsgemässe Mittel eignet sich dadurch für die Verwendung in Haarentfernern, medizinischen Produkten wie zum Beispiel gegen Schuppenflechte oder Neurodermitis, insbesondere zu deren Prävention, weiterhin für Haarfärbeprodukte und für die Verwendung im Intimbereich.

In einer anderen bevorzugten Ausführungsform wird die Zubereitung bei alkalischem pH zwischen 7 und 14 eingesetzt, bevorzugt zwischen 8 und 12 eingesetzt. Typische Beispiele für die Verwendungen bei alkalischem pH sind Rasierseife, Haarfärbemittel, Hautreinigung auf Seifenbasis.

Die erfindungsgemässen Zubereitungen werden als oder für die Herstellung von Reinigungs- und Pflegemitteln verwendet, bevorzugt als kosmetisches, dermatologische oder medizinisches Reinigungs- und/oder Pflegemittel, welches direkt oder indirekt mit dem menschlichen Körper in Berührung kommt. Dies sind beispielsweise: Duschzubereitungen, Badezusätze, Handseifen, auch zur Grobreinigung, Handpasten, Intimreinigung, Reinigung des Augenbereichs, Makeup-Entferner, Augenmakeup-Entferner, Körperreinigung, Mund- und Zahnpflege, Haarshampoos, konditionierende Shampoos, Antischuppen-Shampoo, Babyshampoo, Reinigungs- und Pflegeprodukte bei Schuppenflechte, Neurodermitis, Akne; insbesondere für die Prävention, auch mit pflegenden oder anderen Wirkstoffen, z.B. desodorierend, anti-ageing u.a., Haarentfernung und Rasierprodukte, Haar-Tonicen, Haarfärbeshampoos, Haarfärbemittel und Haarstyling-Zubereitungen, Mundpflegeprodukt (Mundhygieneprodukt), insbesondere in Form von Zahnpasta, Zahncreme, Zahngel, Zahnpulver, Zahnputzflüssigkeit, Zahnputzschaum, Mundwasser, Mundwasserkonzentrat, Zahncreme und Mundwasser als 2-in-1 Produkt, Mundspray. Eingeschlossen ist erfindungsgemäss die Tierpflege und -reinigung.

Eine bevorzugte Ausführungsvariante der Zubereitungen findet Verwendung als Shampoo, Duschgel, und Handseife.

Eine weitere bevorzugte Ausführungsvariante der Zubereitungen findet Verwendung zur Haarentfernung und Rasur.

Eine weitere bevorzugte Ausführungsvariante der Zubereitungen findet Verwendung als Mundpflegeprodukte.

Im Sinne dieser Anmeldung kann das Mittel als Flüssigkeit, Lösung oder Dispersion, Emulsion, Lotion, Gel, Spray oder Schaum verwendet werden. So können sie z. B. eine Lösung, eine Emulsion (O/W), oder eine multiple Emulsion, beispielsweise vom Typ Wasser-in-ÖI-in-Wasser (W/O/W), ein Gel, eine Hydrodispersion, eine lamellare Phase, eine flüssige isotrope Lösungsphase oder eine micellare Phase, darstellen.

Bevorzugt ist die flüssige oder gelförmige Ausführungsform mit Wasser oder einem organischen Lösungsmittel, ganz besonders bevorzugt ist die wässrige Ausführungsform.

Die Zubereitungen eignen sich hierbei sowohl zur verdünnten Anwendung, als auch zur direkten Applikation als auch zur Anwendung über ein Hilfsmittel.

Sie können als auf der Haut und dem Haar verbleibende ("leave-on") oder abzuspülende ("rinse-off") Produkte formuliert werden. In einer besonders bevorzugten Ausführungsform handelt es sich bei den erfindungsgemässen Zubereitungen um Rinse-off-Formulierungen. Moderne Rinse-off Produkte haben häufig einen hohen Anteil an konditionierenden Wirkstoffen, die auch aus Ölanteilen bestehen können. Folglich können diese Zubereitungen als Emulsionen vorliegen.

Alle Sachverhalte, Gegenstände und Ausführungsformen, die für die Zubereitung beschrieben sind, sind auch auf das Reinigungs-und Pflegeverfahren sowie deren Verwendung anwendbar und umgekehrt.

Weitere Aspekte der vorliegenden Erfindung ergeben sich aus den nachfolgenden Beispielen sowie den beigefügten Patentansprüchen.

### Vollständige oder teilweise Substitution von mittelkettigen Tensiden (II) durch Verbindung (I)

In den Versuchen ist Verbindung (I) Sodium PEG-7-olive oil carboxylate, Referenz C12 : Sodium Laureth-5 carboxylate, Referenz Oleyl: Sodium Oleth-6 carboxylate, SLES = Sodium laureth sulfate;

Da die Reinigungskraft der hier betrachteten Reinigungs-und Pflegemittel massgeblich durch die darin enthaltenden Tenside bestimmt wird, werden die Vergleichsversuche auf Basis der wasserbasierten Tensidsysteme ohne die weiteren Zusatzstoffe durchgeführt. Exemplarisch werden die Untersuchungen für die Mittel mit Sodium laureth sulfate gezeigt.

### a) Verbessertes Schmutztragevermögen bei Substitution von SLES durch Verbindung (I)

Das Schmutztragevermögen wird als 2%-ige Lösung gegenüber Sodium laureth sulfate und Referenzen gemessen, die Trendlinie für die Sedimentation in Wasser ist eingezeichnet.

Ergebnis: Überraschenderweise zeigt Verbindung (I) das gleiche gute Schmutztragevermögen wie SLES.

Die strukturanaloge Oleyl-Referenz (keine Mischung von Fettsäureketten) und die Referenz mit gesättigter C12-Kette (üblicherweise eingesetzte Tenside) zeigen ein deutlich schlechteres Tragevermögen. Verbindung (I) kann überraschenderweise SLES substituieren, im Gegensatz zu analogen Verbindungen von (I) auf Basis von mittelkettigen Fettsäuren oder gereinigten längerkettigen Fettsäuren.

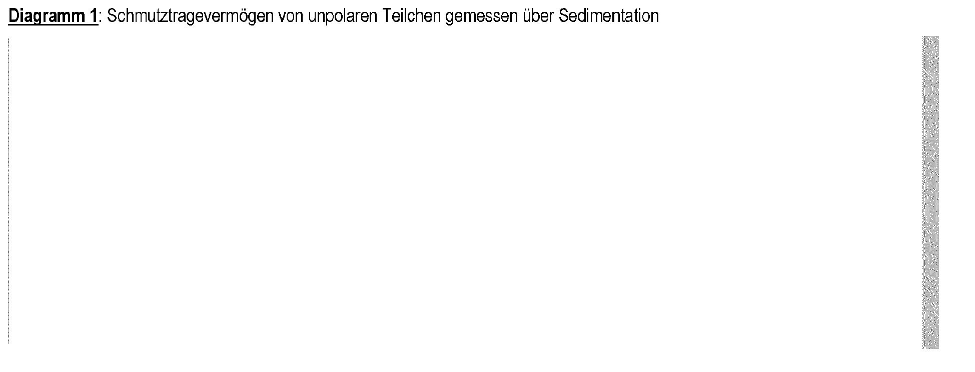

### Verhalten in hartem Wasser:

Bei Zugabe von CaCl₂ zu der Dispersion bricht der Schaum von SLES zusammen; die Teilchen flocken aus, das Schmutztragevermögen geht verloren. Dahingegen bleiben in den Mitteln enthaltend Verbindung (I) unerwarteterweise der Schaum erhalten und das Schmutztragevermögen bleibt intakt. Diese Mittel bilden somit auch einen guten Schaum in hartem und elektrolythaltigem Wasser, z.B. in Salzwasser- Shampoos.

### (b) Bestimmung der optimalen Konzentrationsverhältnissen von Verbindung (I) zu mittelkettigen Tenside (II)

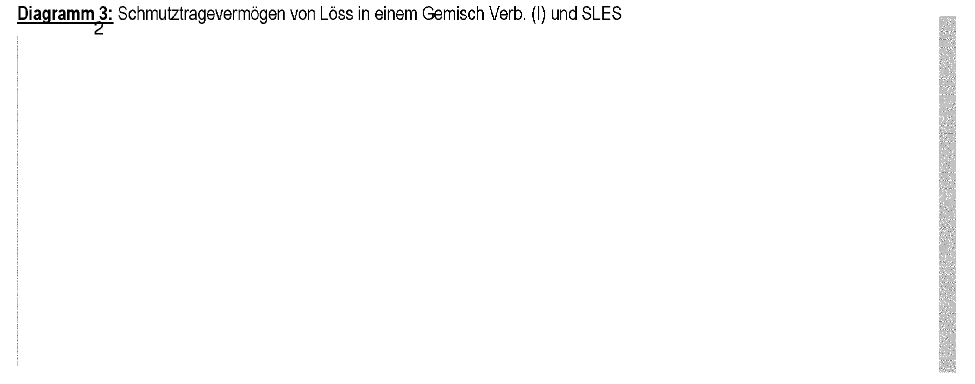

**Ergebnis:** Ein erster Effekt auf die Sedimentationsgeschwindigkeit ist ab einem Verhältnis von Verb. (I): SLES = 1:1 zu erkennen. Bei einer nur teilweisen Substition von SLES, wird das beste Schmutztragevermögen wird bei den Verhältnissen Verb.(I) : SLES ≥ 3,5 erzielt.

### Hartes Wasser/ Hoher Elektrolytgehalt:

Das gute Schmutztragevermögen der Mischungen wird auch bei Zugabe von CaCl₂ erhalten.

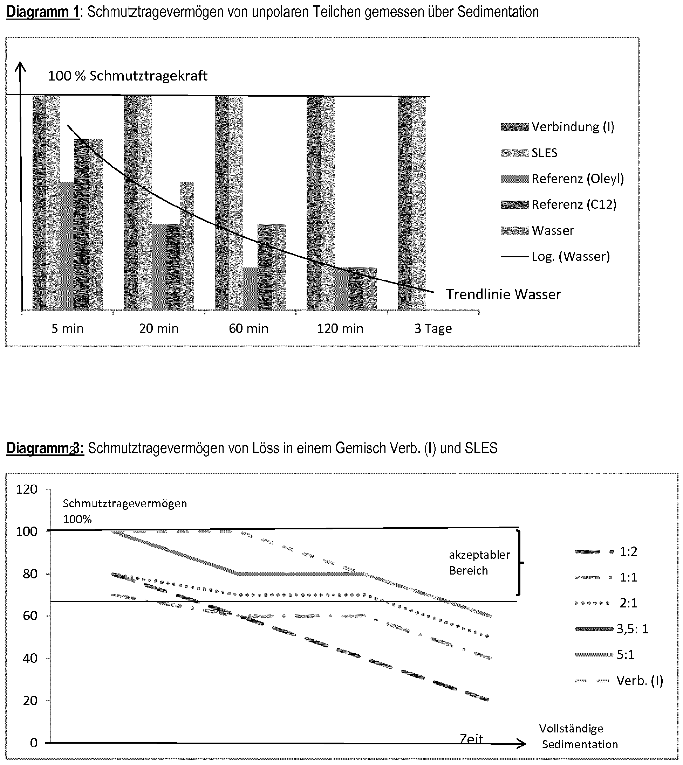

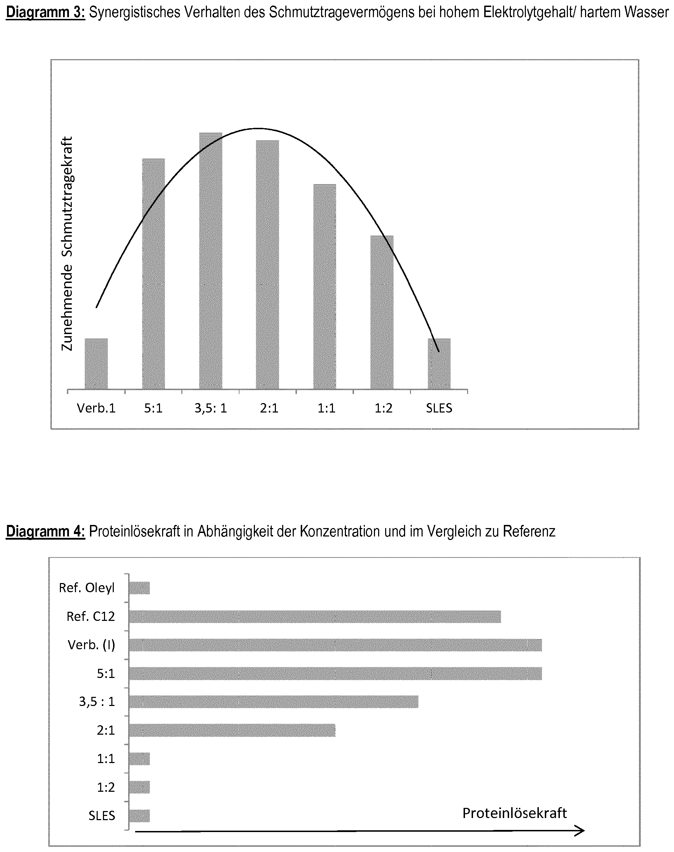

Überraschenderweise zeigen die Mischungen hier ein synergistisches Verhalten (Diagramm 3) - das Schmutztragevermögen der Mischungen ist deutlich höher als für die Lösungen der Einzelverbindungen (I) oder SLES.

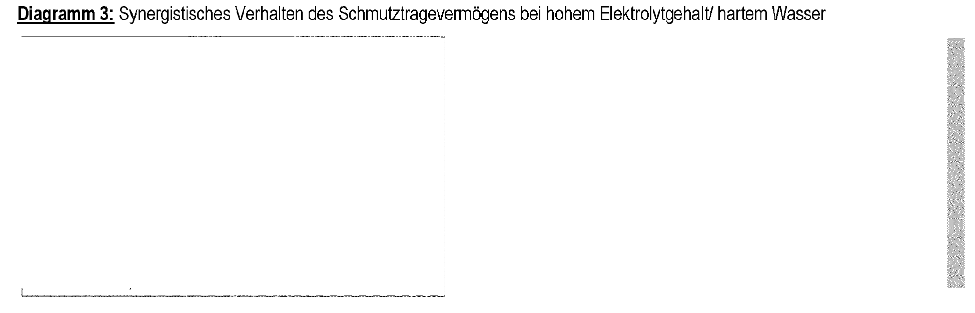

### Schaum:

Schaumbildung und Schaumstabilität verhalten sich in den Mitteln additiv. Damit ist ein gutes Schaumverhalten gewährleistet. In hartem Wasser bleibt der Schaum nur bei den beschriebenen Mitteln stabil.

### (c) Verbesserte Proteinlösekraft bei vollständiger oder teilweiser Substitution von SLES durch Verbindung 1

Die Tests wurden mit 84%-igem eingetrockneten Eiweiss (Mischung aus tierischem und pflanzlichem Protein) und einer 3%-igen Tensidlösung in Wasser durchgeführt.

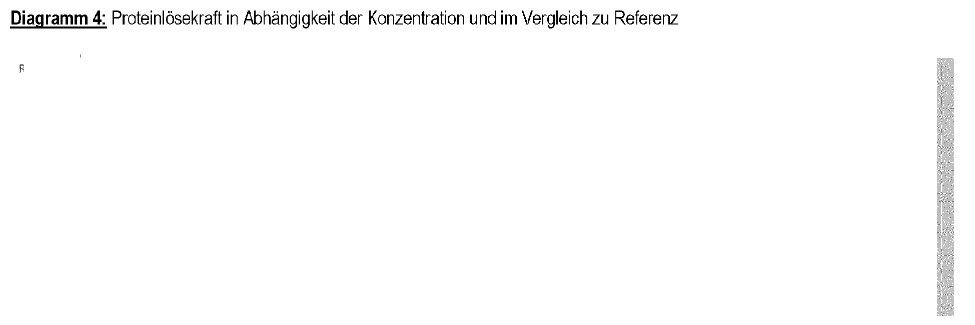

Ergebnis: Die Mittel Verb (I), Verb (I): SLES ≥ 3,5 : 1, bzw. SLES ≥ 5 : 1 weisen die grösste Reinigungskraft auf die Proteinverunreinigungen auf. Analoge Ergebnisse wurden auf Baumwolle und Viskose erhalten.

### (d) Verminderte Augenreizung bei vollständiger oder teilweiser Substitution von SLES durch Verbindung 1

Exemplarisch wird eine repräsentative Konzentration von Tensiden in einer milden Reinigungs- und Pflegezubereitung genommen, analog für Alkylethersulfate, Alkylsulfate, Acylsarcosinate, Alkylglucoside, Amidoalkylbetaine, Alkanolamide und Amphoacetate gemäss Anspruch 1.

### Diagramm 5:

| **Verhältnis Verb.(I)/SLES** | Einstufung |
|---|---|
| SLES | Schwere Augenschäden (Kategorie 1)-irreversibel |
| 1:2 | Augenreizung (Kategorie 2)-reversibel |
| 1:1 | Augenreizung (Kategorie 2)-reversibel |
| 2:1 | Augenreizung (Kategorie 2)- reversibel |
| 3,5:1 | - |
| 5:1 | - |
| Verb. (I) | - |

(Zur Vermeidung von Tierversuchen, wird für diese Einstufung auf die Kategorisierung gemäss Kennzeichungspflicht CLP zugegegriffen)

Ergebnis: Die Zusammensetzung des Tensidsystems enthaltend Verbindung (I) kann die Augenreizung mindestens um eine Kennzeichnungsstufe verringern.

### Zusammenfassung

Durch Substituion von mittelkettigen Tensiden durch Verbindung 1 konnten überraschenderweise deutliche Verbesserungen in folgenden Bereichen erreicht werden:
Dispergiervermögen
Proteinreinigungskraft
Schaumverhalten
Verhalten gegenüber hartem Wasser
Augenreizung
bevorzugt wird ein Verhältnis von (I) zu der Summe der Tenside (II) ≥ 2 : 1 ist, bevorzugt ≥ 3,5 : 1 und besonders bevorzugt ≥ 5 :1.

### Ausführungsbeispiele zu der Kombination von Verbindung (I) mit Tensiden (III), auch in Kombination mit (II)

Verbindung (A) = Ethoxyliertes Fettsäureamid; Verbindung (B) = Polyhydroxy glucamid, Verbindung (C) = Amphoacate (D)= Ethoxylierter Fettsäuremethylester

### (a) Schmutztragevermögen bleibt durch zusätzliche Tenside (III) gleich oder wird besser

Es wird die Sedimentationsgeschwindigkeit von Mineralerde in einer 3%-igen Tensidlösung bestimmt. Die Veränderungen bei Zugabe eines weiteren Tensids zu der reinen Lösung von Verbindung (I) und der Lösung von Verbindung (II) mit SLES (3,5:1) werden bestimmt (Tabelle 1).

**Tabelle 1: Veränderung des Schmutztragevermögens bei Zugabe eines weiteren Tensids**

| | A | B | C | D |
|---|---|---|---|---|
| Verb. (I) | + | n.d. | + | = |
| Verb. (I) + SLES (3,5:1) | + | + | + | = |

| | | | | |
|---|---|---|---|---|
| + Verbesserung durch 3. Tensid - Verschlechterung durch 3. Tensid = 3. Tensid hat keinen Einfluss | | | | |

Ergebnis: Überraschenderweise haben die Tenside A-C aus C-18-Pflanzenölen einen positiven Einfluss auf das Schmutztragevermögen. Das bereits sehr gute Schmutztragevermögen wird durch die erfindungsgemässe Zugabe eines weiteren Tensids A-D nicht beeinträchtigt, im Gegenteil die Tenside A-C aus C-18-Pflanzenölen verbessern das Schmutztragevermögen.

### (b) Proteinreinigungskraft wird durch zusätzliche Tenside (III) nicht beeinträchtigt

Es wird die Reinigungskraft einer 1,5%-igen Tensidlösung auf Proteine bestimmt. Die Reinigungskraft einer Probe, in der ein Teil des Tensidssystems durch das C-18- Tensid (III) ersetzt wurde, wird verglichen mit der Reinigungskraft des Tensids bei gleicher Gesamttensidkonzentration. Die Veränderung der Reinigungskraft ist in Tabelle 2 dokumentiert.

**Tabelle 2: Veränderung der Proteinreinigungskraft bei Zugabe eines weiteren Tensids**

| | A | B | C | D |
|---|---|---|---|---|
| Verb. (I) | = | n.d. | = | = |
| Verb. (I) + SLES (2:1) | + | + | = | = |

| | | | | |
|---|---|---|---|---|
| + Verbesserung durch 3. Tensid - Verschlechterung durch 3. Tensid = 3. Tensid hat keinen Einfluss | | | | |

Ergebnis: Überraschenderweise haben die Tenside A und B aus C-18-Pflanzenölen einen positiven Einfluss auf das Proteinlösungsvermögen. Das bereits sehr gute Proteinlösevermögen wird durch die erfindungsgemässe Zugabe eines weiteren Tensids A-D nicht beeinträchtigt.

### Weitere Ausführungsbeispiele:

| | **1** | **2** | **Ref** | **3** | **Ref** | **Ref** | **Ref** | **Ref** | **4** |
|---|---|---|---|---|---|---|---|---|---|
| Verbindung (I) | 10,0% | 10,0% | 10,0% | 1,50% | 1,50% | 3,00% | 1,50% | 5,00% | 1,50% |
| PEG-7 Olivenölester | | | | | | | | 1,00% | |
| Natrium olivoilamphoacetat | 5,0% | | | | | | | | 1,50% |
| Sunfloweroyl Methylglucamide | | 2,9% | 2,9% | | | | | | |
| Amidet N | | | | 0,50% | 0,50% | | | | |
| Ethoxylierter Rapssäuremethylester | | | | 0,50% | 0,50% | | | | |
| Sodium laureth sulfate | | | | | | | | | |
| Disodium laureth sulfosuccinat | | | 0,9% | | | | | | |
| Coco amido propyl betaine | | | | | 0,29% | | | | |
| Natriumcitrat | | | | 0,10% | | | | | |
| Tetranatrium Glutamate Diacetat | 0,5% | | | | | 0,20% | 0,20% | 0,20% | 0,20% |
| Tetranatrium Iminosuccinat | | 0,5% | 0,5% | | | | | | |
| Alkohol | 2,0% | | | | | | | | |
| Propylenglycol | | 2,0% | 2,0% | | | | | | |
| Xanthan gum | 0,4% | | | | | | | | |
| Panthenol Acetat | 0,001% | | | | | | | | |
| Olea Europea oil | | | | | | 0,20% | 0,20% | 0,20% | 0,20% |
| optional Konservierungsmittel | 0,001% | 0,001% | 0,001% | 0,001% | 0,001% | 0,001% | 0,001% | 0,001% | 0,001% |
| Milchsäure | pH = 5,5 | pH = 5,5 | pH = 5,5 | pH = 5,5 | pH = 5,5 | pH = 5,5 | pH = 5,5 | pH = 5,5 | pH = 5,5 |
| Wasser | ad 100% | ad 100% | ad 100% | ad 100% | ad 100% | ad 100% | ad 100% | ad 100% | ad 100% |

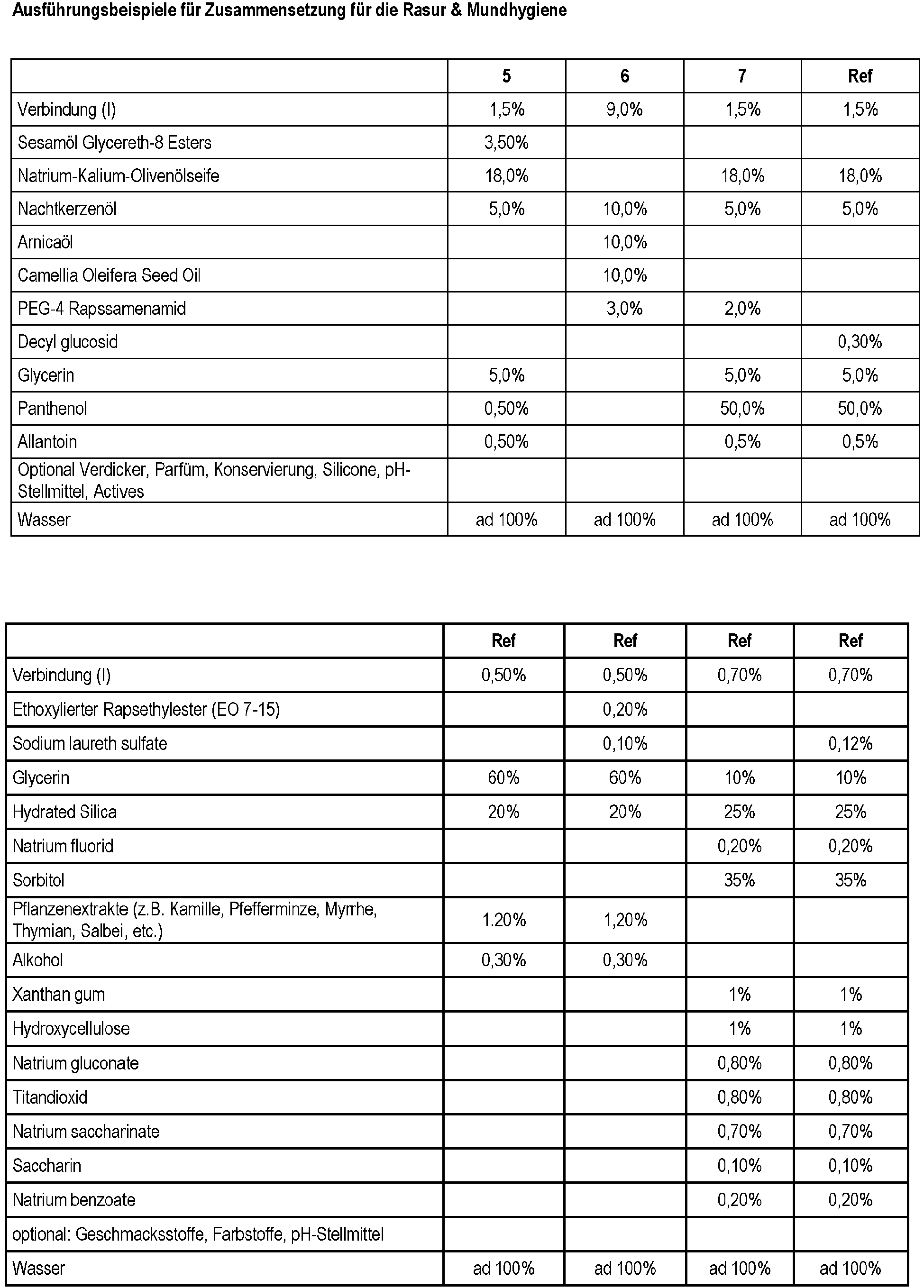

## Patentansprüche

1. Zubereitung als kosmetisches Reinigungs- oder Pflegemittel enthaltend mindestens eine Verbindung der Formel (I)
(I) R'-O(C_{b}H_{2b}O)ₒ-{CH₂-CH[O(C_{b}H_{2b}O)ₚR"]-CH₂O}ᵣ-(C_{b}H_{2b}O)_{q}-R‴
mit **b** einer ganzen Zahl zwischen 2 und 4, **o, p, q** unabhängig voneinander ganzen Zahlen von 0 bis 75, wobei o+p+q mindestens 2 ist, r ist 0 oder 1,
**R', R" und R‴** sind jeweils unabhängig voneinander H, CH₂COOM, oder RCO, wobei einer oder zwei, bevorzugt einer der Reste R', R" und R‴ CH₂COOM darstellen: Wenn R' = CH₂COOM dann gilt o≠0, wenn R" = CH₂COOM dann gilt p≠0, wenn R" - CH₂COOM dann gilt q≠0, mit **M** = H, Alkali-, Erdalkali-, Ammonium-, oder organisches Ammoniumkation, und wobei einer oder zwei der Reste R', R" und R‴ Fettsäureacylreste RCO darstellen; mit **R** einer gesättigten, ein- oder mehrfach ungesättigten Kohlenwasserstoffkette mit 5-23 Kohlenstoffatomen; welche aus einem C-18-Pflanzenöl abgeleitet ist;
wobei der Anteil von 18 und mehr Kohlenstoffatomen der Fettsäureacylreste RCO in der Verbindung (I) oder in dem Gemisch von Verbindungen (I) über 60 Gew.-%, bevorzugt über 72 Gew.-% und ganz besonders bevorzugt von über 77 Gew.-% liegt; und der Anteil der ungesättigten Fettsäureacylresten über 55 Gew.-%, vorzugsweise über 65 Gew.-% und besonders bevorzugt über 72 Gew.-% liegt, jeweils bezogen auf den Gesamtanteil an Fettsäureacylresten RCO der ein oder mehreren Verbindungen (I) im Reinigungs- oder Pflegemittel;
und wobei die Verbindung (I) vorzugsweise aus einem Gemisch unterschiedlicher Kettenlängen und Sättigungsgrade des Fettsäurerest RCO besteht;
und wobei die Polyglykolkette der Verbindung (I) vorzugsweise pflanzlichen Ursprungs ist;
und wobei die Zubereitung optional ein oder mehr zusätzliche Tenside (II) umfasst, ausgewählt aus den Gruppen der Alkylethersulfate, Alkylsulfate, Acylglutamate, Acylsarcosinate, Sulfosuccinatester, Alkylglucoside, Amidoalkylbetaine, Alkanolamide und Amphoacetate, wobei die Tenside gesättigte Alkyl- oder Acylgruppen mit Kettenlängen zwischen 8-18 Kohlenstoffatomen, oder Gemische aus gesättigten Alkyl- oder Acylgruppen mit 8 bis18 Kohlenstoffatomen und ungesättigten C-18-Alkenyl- oder Acylgruppen enthalten, wie sie aus Kokosöl, Palmkernöl oder Babassuöl erhalten werden; und wobei das Verhältnis von (I) zu der Summe der Tenside (II) ≥ 100 : 1 ist;
und wobei die Zubereitung zusätzlich ein oder mehr Tenside (III) umfasst, ausgewählt aus der Gruppe der Seifen, Fettsäureamide und Fettsäureimine und der ethoxylierten Fettsäurealkylester , wobei die Fettsäurereste jeweils von C-18-Pflanzenölen abgeleitet sind und bevorzugt als Mischung gemäss der Fettsäureverteilung im nativen Öl vorliegen oder wie sie bei der Umsetzung von natürlichen Pflanzenölen oder Fetten anfallen;
und wobei die Zubereitung keine Biotensid-Glycolipide aus fermentativer Herstellung enthält.

2. Zubereitung gemäss Anspruch 1 wobei die Summe der Tenside, abgeleitet von C-18-Fettsäuren (I) und (III) bezogen auf die Gesamtmasse der in der Zubereitung vorliegenden Tenside > 50 Gew.-%, bevorzugt > 65 Gew.-%, besonders bevorzugt > 75 Gew.-% beträgt; in einer ganz besonders bevorzugten Ausführungsvariante beträgt die Summe der Tenside abgeleitet von C-18-Fettsäuren, (I) + (III) + weiterer optionaler Tenside (IV), bezogen auf die Gesamtmasse der in der Zubereitung vorliegenden Tenside 100 Gew.-%.

3. Zubereitung gemäss einem der vorstehenden Ansprüche, umfassend ein oder mehrere Tenside ausgewählt aus der Gruppe der alkoxylierten Fettsäureamide der Formel (A)
(A) R- CO-NH-(CₘH₂ₘO)ₙ -H
Wobei **m** die ganze Zahlen 2 oder 3 ist, bevorzugt 2, **n** Zahl im Bereich von 2-10, bevorzugt im Bereich 2-8, ganz besonders bevorzugt 2-4, mit **R** = gesättigte, ein- oder mehrfach ungesättigter Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt; und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (A); und wobei das Tensid (A) vorzugsweise aus einem Gemisch unterschiedlicher Kettenlängen und Sättigungsgrade des Fettsäurerests RCO besteht und wobei RCO abgeleitet ist von einem C-18-Pflanzenöl; und wobei vorzugsweise die Polyglykolkette der Verbindung (A) pflanzlichen Ursprungs ist.

4. Zubereitung gemäss einem der vorstehenden Ansprüche, umfassend ein oder mehrere Tenside ausgewählt aus der Gruppe der Polyhydroxy Fettsäureamide der Formel (B) folgend, 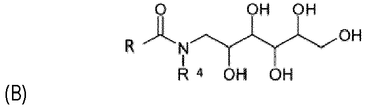
Wobei **R⁴** einen C1-C4-Alkylrest bedeutet, vorzugsweise Methyl und **R** eine gesättigte, ein- oder mehrfach ungesättigter Kohlenwasserstoffkette mit 5-23 Kohlenstoffatomen und RCO abgeleitet aus einem Fettsäuregemisch, wobei der Anteil von 18 und mehr Kohlenstoffatomen des Fettsäurerestes RCO über 60 Gew.-%, bevorzugt über 72 Gew.-% und ganz besonders bevorzugt von über 77 Gew.-% liegt; und wobei der Anteil an ungesättigten Fettsäureresten über 55 Gew.-%, vorzugsweise über 65 Gew.-% und besonders bevorzugt über 72 Gew.-% liegt, jeweils bezogen auf den Gesamtanteil an Fettsäureresten RCO des eingesetzten Tensids (B);
und wobei das Tensid (B) vorzugsweise aus einem Gemisch unterschiedlicher Kettenlängen und Sättigungsgrade des Fettsäurerests RCO wie oben definiert besteht und wobei RCO vorzugsweise abgeleitet ist von einem C-18-Pflanzenöl.

5. Zubereitung gemäss einem der vorstehenden Ansprüche, umfassend ein oder mehrere Tenside ausgewählt aus der Gruppe der Amphoacetate (C), wobei das Tensid (C) vorzugsweise aus einem Gemisch unterschiedlicher Kettenlängen und Sättigungsgrade des Fettsäurerests RCO wie oben definiert besteht und wobei RCO abgeleitet ist von einem C-18-Pflanzenöl.

6. Zubereitung gemäss einem der vorstehende Ansprüche, umfassend ein oder mehrere zusätzliche Tenside ausgewählt aus der Gruppe der alkoxylierten Fettsäureester (D), wobei (D) vorzugsweise aus einem Gemisch unterschiedlicher Kettenlängen und Sättigungsgrade des Fettsäurerests RCO wie definiert in Anspruch 1 besteht und wobei RCO abgeleitet ist von einem C-18-Pflanzenöl; und wobei die Polyglykolkette der Verbindung (D) vorzugsweise pflanzlichen Ursprungs ist;
und wobei die Zubereitung bevorzugt bis 30 Gew.-% einer oder mehrerer Verbindungen (D), bevorzugter bis 20 Gew.- %, besonders bevorzugt bis 10 Gew.-% und ganz besonders bevorzugt 1 Gew.-% bis 3 Gew.-% enthält,
und wobei bevorzugt das Gewichts-Verhältnis von (I) zu der Summe der optional enthaltenen C-18-Pflanzenöl-PEG-7 ester (i), insbesondere bevorzugt zu PEG-7-Olivenölester, ≥ 3,5 : 1 ist, bevorzugt ≥ 5 : 1 und besonders bevorzugt ≥ 9 : 1 ist;
und wobei die Zubereitung bevorzugt bis 5 Gew.-% eines oder mehrerer C-18-Pflanzenöl-PEG-ester (i), bevorzugter bis 3 Gew-%, besonders bevorzugt bis 1 Gew.-% enthält oder in einer bevorzugten Ausführungsform kein zusätzlicher C-18-Pflanzenöl-PEG-ester (i) zu der Verbindung (I) zugegeben wird; Gew.-% bezogen auf die Gesamtmasse der Zubereitung.

7. Zubereitung gemäss einem der vorstehenden Ansprüche, **dadurch gekennzeichnet, dass** sie frei von allen Schwefeltensiden ist.

8. Zubereitung gemäss einem der vorstehenden Ansprüche, **dadurch gekennzeichnet dass** neben dem primären Tensid (I) zusätzlich mindestens vier weitere Inhaltsstoffe umfasst, ausgewählt aus den Gruppen: Lösungsmittel, weitere Tenside, Komplexbildner, Viskositätsregulatoren, pH-Stellmittel und Puffersubstanzen, Konditionierungsmittel, physiologisch aktive Wirkstoffe, Bindemittel und Konsistenzgeber, Ölkörper, Lösungsvermittler, Abrasiva, Antioxidantien, Vitamine, UV-Filter, Trübungsmittel, Konservierungsmittel Duftstoffe, Farbstoffe, anorganische Alkali- oder Erdalkalisalze.

9. Zubereitung gemäss einem der vorhergehenden Ansprüche zur Verbesserung der Reinigungsleistung eines Reinigungs- oder Pflegemittel, insbesondere zur Entfernung von Proteinverunreinigungen.

10. Verwendung der Zubereitung gemäss einem der vorhergehenden Ansprüche 1- 9 zur Entfernung von Protein- oder partikulärer Verschmutzung.

11. Verwendung der Zubereitung gemäss einem der vorhergehenden Ansprüche 1-9 zur Dispergierung von Partikeln in der Zusammensetzung, insbesondere für abrasive Anwendungen.

12. Verwendung der Zubereitung gemäss einem der vorhergehenden Ansprüche 1-9 für einen stabilen Schaum.

13. Nicht-therapeutische Verwendung der Zubereitung gemäss einem der vorhergehenden Ansprüche 1-9 als oder zur Herstellung von Reinigungs- und Pflegemitteln für Körper, Mund und Zähne, Haut und Haare sowie zur Tierpflege.

14. Nicht-therapeutische Verwendung der Zubereitung gemäss einem der vorstehenden Ansprüche 1-9 als oder für die Herstellung von Reinigungs- und Pflegemitteln, welche direkt oder indirekt mit dem menschlichen Körper in Berührung kommen, umfassend Duschzubereitungen, Badezusätze, Handseifen, auch zur Grobreinigung, Handpasten, Intimreinigung, Reinigung des Augenbereichs, Kontaktlinsenreinigung, Makeup-Entferner, Augenmakeup-Entferner, Körperreinigung, Mund- und Zahnpflege, Haarshampoos, konditionierende Shampoos, Antischuppen-Shampoo, Babyshampoo, Reinigungs- und Pflegeprodukte bei Schuppenflechte, Neurodermitis, Akne; insbesondere für die Prävention, auch mit pflegenden oder anderen Wirkstoffen, z.B. desodorierend, anti-ageing u.a., Haarentfernung und Rasierprodukte, Haar-Tonicen, Haarfärbeshampoos, Haarfärbemittel und Haarstyling-Zubereitungen, Mundpflegeprodukt (Mundhygieneprodukt), insbesondere in Form von Zahnpasta, Zahncreme, Zahngel, Zahnpulver, Zahnputzflüssigkeit, Zahnputzschaum, Mundwasser, Mundwasserkonzentrat, Zahncreme und Mundwasser als 2-in-1 Produkt, Mundspray, eingeschlossen ist die Tierpflege und -reinigung.

15. Nicht-therapeutische Verwendung der Zubereitung gemäss einem der vorstehenden Ansprüche 1-9 in Form von Rinse-off-Formulierungen.

16. Nicht-therapeutische Reinigungs-und Pflegeverfahren umfassend die Verfahrensschritte:
a) Bereitstellung einer Zubereitung umfassend ein Reinigungs- oder Pflegemittel gemäss einem der vorstehenden Ansprüche 1-9.
b) in Kontakt bringen von Körper, Zähnen, Zahnfleisch, Haaren oder Fell mit dem Reinigungs- oder Pflegemittel gemäss a).

## Claims

1. Preparation as a cosmetic cleaning or care agent containing at least one compound of the formula (I)
(I) R'-O(C_{b}H_{2b}O)ₒ-{CH₂-CH[O(C_{b}H_{2b}O)ₚR"]-CH₂O}ᵣ-(C_{b}H_{2b}O)_{q}-R‴
wherein b is an integer between 2 and 4, o, p, q are each independently integers from 0 to 75, where o+p+q is at least 2, r is 0 or 1,
R', R" and R‴ are each independently H, CH₂COOM, or RCO, wherein one or two, preferably one, of the radicals R', R" and R‴ are CH₂COOM: if R' = CH₂COOM then o≠0, if R" = CH₂COOM then p≠0, if R‴= CH₂COOM then q≠0, with M = H, alkali metal cation, alkaline earth metal cation, ammonium cation, or organic ammonium cation, and wherein one or two of the radicals R', R", and R‴ are fatty acid acyl radicals RCO; with R a saturated, mono- or polyunsaturated hydrocarbon chain having 5-23 carbon atoms; which is derived from a C-18 vegetable oil;
wherein the proportion of 18 and more carbon atoms of the fatty acid acyl radicals RCO in the compound (I) or in the mixture of compounds (I) is above 60 wt.%, preferably above 72 wt.% and very particularly preferably above 77 wt.%; and the proportion of unsaturated fatty acid acyl radicals is above 55 wt.%, preferably above 65 wt.% and particularly preferably above 72 wt.%, in each case based on the total proportion of fatty acid acyl radicals RCO of the one or more compounds (I) in the cleaning or care agent;
and wherein the compound (I) preferably consists of a mixture of different chain lengths and degrees of saturation of the fatty acid radical RCO;
and wherein the polyglycol chain of compound (I) is preferably of vegetable origin;
and wherein the preparation optionally comprises one or more additional surfactants (II) selected from the groups of alkyl ether sulphates, alkyl sulphates, acyl glutamates, acyl sarcosinates, sulphosuccinate esters, alkyl glucosides, amidoalkyl betaines, alkanolamides and amphoacetates, said surfactants containing saturated alkyl or acyl groups with chain lengths between 8-18 carbon atoms, or mixtures of saturated alkyl or acyl groups with 8 to18 carbon atoms and unsaturated C-18 alkenyl or acyl groups as obtained from coconut oil, palm kernel oil or babassu oil; and wherein the ratio of (I) to the sum of surfactants (II) is ≥ 100 : 1;
and wherein the preparation additionally comprises one or more surfactants (III) selected from the group of soaps, fatty acid amides and fatty acid imines and ethoxylated fatty acid alkyl esters, wherein the fatty acid radicals are each derived from C-18 vegetable oils and are preferably present as a mixture according to the fatty acid distribution in the native oil or as they occur from the conversion of natural vegetable oils or fats;
and wherein the preparation does not contain any glycolipid biosurfactants from fermentative production.

2. Preparation according to claim 1, wherein the sum of the surfactants derived from C-18 fatty acids (I) and (III), based on the total mass of the surfactants present in the preparation, is > 50 wt.%, preferably > 65 wt.%, particularly preferably > 75 wt.%; in a very particularly preferred embodiment, the sum of the surfactants derived from C-18 fatty acids, (I) + (III) + further optional surfactants (IV), is 100 wt.%, based on the total mass of the surfactants present in the preparation.

3. Preparation according to any one of the preceding claims, comprising one or more surfactants selected from the group of alkoxylated fatty acid amides of the formula (A),
(A) R- CO-NH-(CₘH₂ₘO)ₙ -H
wherein m is the integer 2 or 3, preferably 2, n is a number in the range 2-10, preferably in the range 2-8, most preferably 2-4, with R = saturated, mono- or polyunsaturated hydrocarbon chain with 5-23 carbon atoms and RCO derived from a fatty acid mixture, wherein the proportion of 18 and more carbon atoms of the fatty acid radical RCO is above 60 wt.-%, preferably above 72 wt.-%, and particularly preferably above 77 wt.%; and wherein the proportion of unsaturated fatty acid radicals is above 55 wt.%, preferably above 65 wt.% and particularly preferably above 72 wt.%, in each case based on the total proportion of fatty acid radicals RCO of the surfactant (A) used; and wherein the surfactant (A) preferably consists of a mixture of different chain lengths and degrees of saturation of the fatty acid radical RCO and wherein RCO is derived from a C-18 vegetable oil; and wherein preferably the polyglycol chain of compound (A) is of vegetable origin.

4. Preparation according to any one of the preceding claims, comprising one or more surfactants selected from the group of polyhydroxy fatty acid amides following the formula (B), 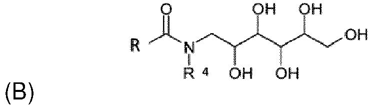
wherein R⁴ is a C1-C4 alkyl radical, preferably methyl, and R is a saturated, mono- or polyunsaturated hydrocarbon chain having 5-23 carbon atoms, and RCO is derived from a fatty acid mixture, wherein the proportion of 18 and more carbon atoms of the fatty acid radical RCO is above 60 wt.%, preferably above 72 wt.% and particularly preferably above 77 wt.%; and wherein the proportion of unsaturated fatty acid radicals is above 55 wt.%, preferably above 65 wt.% and particularly preferably above 72 wt.%, in each case based on the total proportion of fatty acid radicals RCO of the surfactant (B) used;
and wherein the surfactant (B) preferably consists of a mixture of different chain lengths and degrees of saturation of the fatty acid radical RCO as defined above and wherein RCO is preferably derived from a C-18 vegetable oil.

5. Preparation according to any one of the preceding claims, comprising one or more surfactants selected from the group of amphoacetates (C), wherein the surfactant (C) preferably consists of a mixture of different chain lengths and degrees of saturation of the fatty acid radical RCO as defined above and wherein RCO is derived from a C-18 vegetable oil.

6. Preparation according to any one of the preceding claims, comprising one or more additional surfactants selected from the group of alkoxylated fatty acid esters (D), wherein (D) preferably consists of a mixture of different chain lengths and degrees of saturation of the fatty acid radical RCO as defined in claim 1 and wherein RCO is derived from a C-18 vegetable oil; and wherein the polyglycol chain of compound (D) is preferably of vegetable origin;
and wherein the preparation preferably contains up to 30 wt.% of one or more compounds (D), more preferably up to 20 wt.%, and particularly preferably up to 10 wt.%, and very particularly preferably 1 wt.% to 3 wt.%,
and wherein preferably the weight ratio of (I) to the sum of the optionally contained C-18 vegetable oil PEG-7 esters (i), particularly preferably to PEG-7 olive oil esters, is ≥ 3.5 : 1, preferably ≥ 5 : 1 and particularly preferably ≥ 9 : 1;
and wherein the preparation preferably contains up to 5 wt.% of one or more C-18 vegetable oil PEG esters (i), more preferably up to 3 wt.%, particularly preferably up to 1 wt.%, or in a preferred embodiment no additional C-18 vegetable oil PEG ester (i) is added to compound (I); wt.% based on the total mass of the preparation.

7. Preparation according to any one of the preceding claims, **characterized in that** it is free from all sulphur surfactants.

8. Preparation according to any one of the preceding claims, **characterized in that**, it additionally comprises at least four further ingredients besides the primary surfactant (I), selected from the groups: Solvents, further surfactants, complexing agents, viscosity regulators, pH-adjusting agents and buffer substances, conditioning agents, physiologically active agents, binders and consistency agents, emollients, solubilisers, abrasives, antioxidants, vitamins, UV filters, opacifiers, preservatives, fragrances, dyes, inorganic alkali metal salts or alkaline earth metal salts.

9. Preparation according to any one of the preceding claims for improving the cleaning performance of a cleaning or care agent, in particular for removing protein impurities.

10. Use of the preparation according to any one of the preceding claims 1- 9 for the removal of protein or particulate contamination.

11. Use of the preparation according to any one of the preceding claims 1-9 for dispersing particles in the composition, in particular for abrasive applications.

12. Use of the preparation according to any one of the preceding claims 1-9 for stable foam.

13. Non-therapeutic use of the preparation according to any one of the preceding claims 1-9 as or for the preparation of cleaning and care agents for body, mouth and teeth, skin and hair as well as for animal care.

14. Non-therapeutic use of the preparation according to any one of the preceding claims 1-9 as or for the preparation of cleaning and care agents which come into direct or indirect contact with the human body, comprising shower preparations, bath additives, hand soaps, also for coarse cleaning, hand pastes, intimate cleansing, cleansing of the eye area, contact lens cleansing, make-up remover, eye make-up remover, body cleansing, oral and dental care, hair shampoos, conditioning shampoos, anti-dandruff shampoo, baby shampoo, cleansing and care products for psoriasis, neurodermatitis, acne; especially for prevention, also with nourishing or other active ingredients, e.g. deodorising, anti-ageing, etc., hair removal and shaving products, hair tonics, hair colouring shampoos, hair dyeing products and hair styling preparations, oral care product (oral hygiene product), in particular in the form of toothpaste, tooth creme, tooth gel, tooth powder, tooth brushing liquid, tooth brushing foam, mouthwash, mouthwash concentrate, toothpaste and mouthwash as a 2-in-1 product, mouth spray, including animal care and cleaning.

15. Non-therapeutic use of the preparation according to any of the preceding claims 1-9 in the form of rinse-off formulations.

16. Non-therapeutic cleaning and care method comprising the process steps of:
(a) providing a preparation comprising a cleaning or care agent according to any one of the preceding claims 1-9
b) contacting the body, teeth, gums, hair or fur with the cleaning or care composition according to a).

## Revendications

1. Préparation en tant que produit cosmétique de nettoyage ou de soin contenant au moins un composé de formule (I)
(I) R'-O(C_{b}H_{2b}O)ₒ-{CH₂-CH[O(C_{b}H_{2b}O)ₚR"]-CH₂O}ᵣ-(C_{b}H_{2b}O)_{q}-R‴
avec b un entier compris entre 2 et 4, o, p, q indépendamment l'un de l'autre des entiers de 0 à 75, où o+p+q est au moins 2, r est 0 ou 1,
R', R" et R‴ sont chacun indépendamment H, CH₂COOM, ou RCO, où un ou deux, de préférence un, des radicaux R', R" et R‴ représentent CH₂COOM: Si R' = CH₂COOM alors o≠0, si R" = CH₂COOM alors p≠0, si R‴= CH₂COOM alors q≠0, avec M = H, cation alcalin, alcalino-terreux, ammonium ou ammonium organique, et où un ou deux des radicaux R', R" et R‴ représentent des radicaux acyle d'acide gras RCO; avec R une chaîne hydrocarbonée saturée, mono ou polyinsaturée, ayant de 5 à 23 atomes de carbone; qui est dérivée d'une huile végétale C-18;
où la proportion de 18 atomes de carbone et plus des radicaux acyle d'acide gras RCO dans le composé (I) ou dans le mélange de composés (I) est supérieure à 60 % en poids, de préférence supérieure à 72 % en poids et de manière tout particulièrement préférée supérieure à 77 % en poids; et la proportion des radicaux acyle d'acide gras insaturés est supérieure à 55 % en poids, de préférence supérieure à 65 % en poids et de manière particulièrement préférée supérieure à 72 % en poids, dans chaque cas par rapport à la proportion totale de radicaux acyle d'acide gras RCO dans l'un ou dans les plusieurs composés (I) dans le produit de nettoyage ou de soin;
et où le composé (I) est de préférence constitué d'un mélange de différents longueurs de chaîne et degrés de saturation du radical d'acide gras RCO;
et où la chaîne de polyglycol du composé (I) est de préférence d'origine végétale;
et où la préparation comprend optionnellement un ou plusieurs agents tensioactifs supplémentaires (II) choisis dans les groupes des alkyléthersulfates, alkylsulfates, acylglutamates, acylsarcosinates, esters de sulfosuccinate, alkylglucosides, amidoalkylbétaïnes, alcanolamides et amphoacétates, les agents tensioactifs contenant des groupes alkyle ou acyle saturés ayant des longueurs de chaîne comprises entre 8 et 18 atomes de carbone, ou des mélanges de groupes alkyle ou acyle saturés ayant de 8 à 18 atomes de carbone et de groupes alcényle ou acyle insaturés de 18 atomes de carbone, tels qu'ils sont obtenus à partir d'huile de coco, d'huile de palmiste ou d'huile de babassu; et le rapport de (I) à la somme des agents tensioactifs (II) étant ≥ 100 : 1 ;
et où la préparation comprend en outre un ou plusieurs agents tensioactifs (III), choisis dans le groupe des savons, des amides d'acides gras et des imines d'acides gras et des esters alkyliques d'acides gras éthoxylés, les radicaux d'acides gras étant à chaque fois dérivés d'huiles végétales C-18 et se présentant de préférence sous forme de mélange selon la distribution des acides gras dans l'huile native ou tels qu'ils apparaissent lors de la conversion d'huiles végétales naturelles ou de graisses végétales;
et où la préparation ne contient pas de bio-tensioactifs de glycolipides issus d'une production par fermentation.

2. Préparation selon la revendication 1, dans laquelle la somme des agents tensioactifs dérivés d'acides gras C-18 (I) et (III) par rapport à la masse totale des agents tensioactifs présents dans la préparation est > 50 % en poids, de préférence > 65 % en poids, de manière particulièrement préférée > 75 % en poids; dans une variante de réalisation tout à fait préférée, la somme des agents tensioactifs dérivés d'acides gras C-18, (I) + (III) + d'autres agents tensioactifs optionnels (IV), par rapport à la masse totale des agents tensioactifs présents dans la préparation, est de 100 % en poids.

3. Préparation selon l'une quelconque des revendications précédentes, comprenant un ou plusieurs agents tensioactifs choisis dans le groupe des amides d'acides gras alcoxylés de formule (A)
(A) R-CO-NH-(CₘH₂ₘO)ₙ-H
dans laquelle m est le nombre entier 2 ou 3, de préférence 2, n est un nombre compris entre 2 et 10, de préférence entre 2 et 8, tout particulièrement entre 2 et 4, avec R = chaîne hydrocarbonée saturée, mono- ou polyinsaturée avec 5 à 23 atomes de carbone et RCO dérivé d'un mélange d'acides gras, où la proportion de 18 atomes de carbone et plus du radical d'acide gras RCO est supérieure à 60 % en poids, de préférence supérieure à 72 % en poids et tout particulièrement préférée supérieure à 77 % en poids; et où la proportion de radicaux d'acides gras insaturés est supérieure à 55 % en poids, de préférence supérieure à 65 % en poids et de manière particulièrement préférée supérieure à 72 % en poids, dans chaque cas par rapport à la proportion totale de radicaux d'acides gras RCO de l'agent tensioactif (A) utilisé; et où l'agent tensioactif (A) est de préférence constitué d'un mélange de différents longueurs de chaîne et degrés de saturation du radical d'acide gras RCO et où RCO est dérivé d'une huile végétale C-18; et où de préférence la chaîne de polyglycol du composé (A) est d'origine végétale.

4. Préparation selon l'une quelconque des revendications précédentes, comprenant un ou plusieurs agents tensioactifs choisis dans le groupe des amides d'acides gras polyhydroxylés de formule (B) suivante, 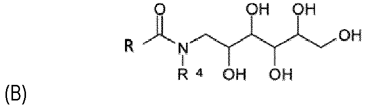 dans laquelle R⁴ est un radical alkyle en C1-C4, de préférence méthyle, et R est une chaîne hydrocarbonée saturée, mono- ou polyinsaturée ayant de 5 à 23 atomes de carbone et RCO issu d'un mélange d'acides gras, la proportion de 18 atomes de carbone et plus du radical d'acide gras RCO étant supérieure à 60 % en poids, de préférence supérieure à 72 % en poids et tout particulièrement préférée supérieure à 77 % en poids; et dans laquelle la proportion de radicaux d'acides gras insaturés est supérieure à 55 % en poids, de préférence supérieure à 65 % en poids et de manière particulièrement préférée supérieure à 72 % en poids, dans chaque cas par rapport à la proportion totale de radicaux d'acides gras RCO du tensioactif (B) utilisé; et où l'agent tensioactif (B) est constitué de préférence d'un mélange de différents longueurs de chaîne et degrés de saturation du radical d'acide gras RCO tel que défini ci-dessus et où RCO est de préférence dérivé d'une huile végétale C-18.

5. Préparation selon l'une quelconque des revendications précédentes, comprenant un ou plusieurs tensioactifs choisis dans le groupe des amphoacétates (C), le tensioactif (C) étant de préférence constitué d'un mélange de différents longueurs de chaîne et degrés de saturation du radical d'acide gras RCO tel que défini ci-dessus, et le RCO étant dérivé d'une huile végétale C-18.

6. Préparation selon l'une quelconque des revendications précédentes, comprenant un ou plusieurs tensioactifs supplémentaires choisis dans le groupe des esters d'acides gras alcoxylés (D), dans laquelle (D) consiste de préférence en un mélange de différents longueurs de chaîne et degrés de saturation du radical d'acide gras RCO tel que défini dans la revendication 1 et dans laquelle RCO est dérivé d'une huile végétale C-18; et dans laquelle la chaîne polyglycol du composé (D) est de préférence d'origine végétale;
et où la préparation contient de préférence jusqu'à 30 % en poids d'un ou plusieurs composés (D), de préférence jusqu'à 20 % en poids, de manière particulièrement préférée jusqu'à 10 % en poids et de manière tout à fait préférée de 1 % en poids à 3 % en poids,
et où, de préférence, le rapport pondéral de (I) à la somme des esters de PEG-7 d'huile végétale C-18 (i) éventuellement contenus, en particulier de préférence aux esters de PEG-7 d'huile d'olive, est ≥ 3,5 : 1, de préférence ≥ 5 : 1 et de manière particulièrement préférée ≥ 9 : 1;
et où la préparation contient de préférence jusqu'à 5 % en poids d'un ou plusieurs esters de PEG d'huile végétale C-18 (i), de préférence jusqu'à 3 % en poids, de manière particulièrement préférée jusqu'à 1 % en poids ou, dans une forme de réalisation préférée, aucun ester de PEG d'huile végétale C-18 (i) supplémentaire n'est ajouté au composé (I); % en poids par rapport à la masse totale de la préparation.

7. Préparation selon l'une quelconque des revendications précédentes, **caractérisée en ce qu'**elle est exempte de tous les tensioactifs soufrés.

8. Préparation selon l'une quelconque des revendications précédentes, **caractérisée en ce que**, outre l'agent tensioactif primaire (I), elle comprend en outre au moins quatre autres ingrédients, choisis dans les groupes: Solvants, autres agents tensioactifs, agents complexants, régulateurs de viscosité, régulateurs de pH et substances tampons, agents de conditionnement, principes physiologiquement actifs, liants et agents de consistance, émollients, agents de solubilisation, abrasifs, antioxydants, vitamines, filtres UV, opacifiants, agents de conservation, parfums, colorants, sels inorganiques alcalins ou alcalino-terreux.

9. Préparation selon l'une quelconque des revendications précédentes pour améliorer la performance de nettoyage d'un produit de nettoyage ou de soin, en particulier pour éliminer les impuretés protéiques.

10. Utilisation de la préparation selon l'une quelconque des revendications précédentes 1-9 pour l'élimination de salissures protéiniques ou particulaires.

11. Utilisation de la préparation selon l'une quelconque des revendications précédentes 1-9 pour disperser des particules dans la composition, en particulier pour des applications abrasives.

12. Utilisation de la préparation selon l'une quelconque des revendications précédentes 1-9 pour obtenir une mousse stable.

13. Utilisation non thérapeutique de la préparation selon l'une quelconque des revendications précédentes 1-9 comme ou pour la fabrication de produits de nettoyage et de soins pour le corps, la bouche et les dents, la peau et les cheveux, ainsi que pour les soins aux animaux.

14. Utilisation non thérapeutique de la préparation selon l'une quelconque des revendications précédentes 1-9 comme ou pour la fabrication de produits de nettoyage et de soins qui entrent en contact direct ou indirect avec le corps humain, comprenant des préparations pour la douche, des additifs pour le bain, des savons pour les mains, également pour le nettoyage grossier, pâtes pour les mains, nettoyage intime, nettoyage de la zone oculaire, nettoyage des lentilles de contact, démaquillant, démaquillant pour les yeux, nettoyage du corps, soins buccaux et dentaires, shampooings capillaires, shampooings conditionnants, shampooings antipelliculaires, shampooings pour bébés, produits de nettoyage et de soins pour le psoriasis, la névrodermite, l'acné; en particulier pour la prévention, également avec des principes actifs traitants ou autres, p. ex. désodorisant, anti-âge, etc., épilation et produits de rasage, toniques capillaires, shampooings colorants pour cheveux, teintures capillaires et préparations de coiffage, produit de soins buccaux (produit d'hygiène buccale), notamment sous forme de dentifrice, pâte dentifrice, gel dentaire, poudre dentaire, liquide de brossage dentaire, mousse de brossage dentaire, bain de bouche, concentré de bain de bouche, dentifrice et bain de bouche sous forme de produit 2 en 1, spray buccal, y compris les soins et le nettoyage des animaux.

15. Utilisation non thérapeutique de la préparation selon l'une quelconque des revendications précédentes 1-9 sous la forme de formulations de rinçage.

16. Procédés de nettoyage et de soins non thérapeutiques comprenant les étapes de procédé :
a) mise à disposition d'une préparation comprenant un produit de nettoyage ou de soin selon l'une quelconque des revendications précédentes 1-9.
b) Mise en contact du corps, des dents, des gencives, des cheveux ou de la fourrure avec le produit de nettoyage ou de soin selon a).
